# EUROPEAN PATENT APPLICATION

(11) **EP 4 803 515 A1**
(43) Date of publication of application: **09.09.2026**
(21) Application number: 24884777.4
(22) Date of filing: 30.10.2024
(51) Int. Cl.: C07D 405/14, C07D 401/14, A61K 31/454, A61K 31/4709, A61P 35/00

(54) **PHTHALAZINONE GSPT1 PROTEIN DEGRADING AGENT AND USE THEREOF**

(30) Priority: 31.10.2023 CN 202311429738
(71) Applicant: China Pharmaceutical University, Nanjing, Jiangsu 211198 (CN); Ascentage Pharma (Suzhou) Co Ltd, Suzhou, Jiangsu 215000 (CN)
(72) Inventor: XU, Xi, Nanjing, Jiangsu 211198 (CN); LI, Zhiyu, Nanjing, Jiangsu 211198 (CN); ZHANG, Yanqing, Nanjing, Jiangsu 211198 (CN); CHANG, Xiujin, Nanjing, Jiangsu 211198 (CN); QU, Fangui, Nanjing, Jiangsu 211198 (CN); BIAN, Jinlei, Nanjing, Jiangsu 211198 (CN); WANG, Jubo, Nanjing, Jiangsu 211198 (CN); QIU, Zhixia, Nanjing, Jiangsu 211198 (CN); WU, Hongxi, Nanjing, Jiangsu 211198 (CN); FAN, Zhongpeng, Nanjing, Jiangsu 211198 (CN); WANG, Xinhong, Nanjing, Jiangsu 211198 (CN); LIU, Wenjing, Nanjing, Jiangsu 211198 (CN)
(74) Representative: Murgitroyd & Company
(86) International application number: PCT/CN2024/128337
(87) International publication number: WO 2025/092775

(57) **Abstract**

Disclosed is a compound as shown in general formula I or a pharmaceutically acceptable salt, tautomer, mesomer, racemate, enantiomer, and diastereomer thereof. The compound of the present invention significantly improves the degradation effect of GSPT1 protein and the antiproliferative inhibition activity of AML and solid tumor cells. Therefore, the compound of the present invention can be used in the preparation of a medicament for treating or preventing diseases related to GSPT1, IKZF1, IKZF2, IKZF3, CK1α, N-MYC or C-MYC protein mutation, expression imbalance, and allostery and functional abnormalities; in addition, said compound can be used to prepare a GSPT1 degrading agent.

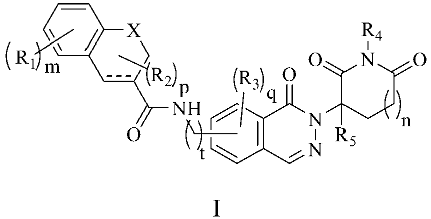

## Description

### TECHNICAL FIELD

The present disclosure pertains to the technical field of pharmaceuticals and particularly relates to a phthalazinone GSPT1 protein degrader and use thereof.

### BACKGROUND

Target protein degradation (TPD) technology is an emerging technology that utilizes a protein homeostasis-regulating protein degradation mechanism inherent in eukaryotic cells to interfere with protein functions. The rise of this technology solves, to a certain extent, the challenges confronting small-molecule inhibitors and gene interference technology. At present, TPD degrades target proteins mainly through ubiquitin proteasomes and lysosomes. Currently, the most developed and studied approaches in this area are proteolysis-targeting chimeras (PROTACs) and molecular glues (MGs), which are based on the ubiquitin-proteasome system. Target protein degradation technology has considerable promise for application and potential for development in a number of disease areas, such as malignancies, neurodegenerative diseases, and metabolic diseases.

Molecular glues are a class of proximity-inducing small molecules that enable precise temporal control over various biological processes, such as signal transduction, transcription, chromatin regulation, and protein folding, localization, and degradation. As chemical inducers of proximity, they can induce protein-protein interactions between E3 ubiquitin ligases and target proteins, leading to the degradation of the latter. Through the formation of ternary complexes, molecular glues can promote the dimerization or co-localization of two proteins, thereby producing a variety of biological and pharmacological functions.

G1 to S phase transition 1 (GSPT1, also known as eRF3a) is a translation termination factor in the body. Downregulation of GSPT1 can lead to abnormal expression of key proteins and inhibit proliferation or induce apoptosis in various tumor cells. Since the discovery that GSPT1 is a new substrate of the E3 ubiquitin ligase CRBN, it has been able to be degraded by molecular glue degraders in a targeted manner. Currently, a variety of molecular glue degraders are undergoing clinical and preclinical research. These molecules are very effective in treating hematological tumors and certain solid tumors. GSPT1 is a multifunctional protein. In addition to its role as a class II peptide chain release factor involved in protein translation termination, it is also related to processes such as cell apoptosis, cell cycle regulation, and tumor development and progression. For example, during cell apoptosis, GSPT1 is processed proteolytically to form another isoform that contains a conserved N-terminal apoptosis-inhibiting protein conjugate. GSPT1 interacts with inhibitors of apoptosis proteins (IAPs) through this form. This interaction relieves the inhibition of IAPs, leading to the release of caspases and thus the promotion of cell apoptosis. Moreover, the M domain of eRF3 interacts with polya-binding protein (PABP), coupling translation termination with mRNA degradation.

Acute myeloid leukemia (AML) is a genetically and biologically heterogeneous myeloid malignancy characterized by the abnormal proliferation of immature myeloid progenitor cells, which disrupts normal hematopoiesis, leading to severe infections, anemia, and bleeding. AML is characterized by poor survival rates and high recurrence rates. Therefore, as a relatively rare malignancy, AML has been considered an orphan disease by the U.S. Food and Drug Administration. GSPT1, as a new substrate of CRL4-CRBN-E3 ubiquitin ligase, plays an important role in treating AML. GSPT1-targeting clinical drugs for treating AML, such as CC-90009 and BTX-1188, are currently under development. CC-90009 is administered mainly by intravenous injection to treat AML, and BTX-1188 is a drug that can be orally administered to treat AML and NHL. Further studies show that the degradation of GSPT1 is associated with upregulated expression of the ATF3 and ATF4 genes, which are critical to the integrated stress response pathway. The activation of the integrated stress response pathway is closely linked to the phosphorylation of eRF2. When activated, the integrated stress response pathway can lead to acute apoptosis of cells.

According to the World Health Organization, breast cancer is the leading cause of malignancy-related death among women. In 2018, Wang et al. analyzed gene expression in the total RNA extracted from breast cancer samples they had collected, and identified five genes, including GSPT1, as potential therapeutic targets for triple-negative breast cancer. Recently, a study by Malta-Vacas et al. revealed that the longer GSPT1 allele 12-GGC was present in 5.1% of breast cancer patients, and mRNA quantification experiments further indicated that GSPT1 was overexpressed in tumor tissues from patients with the allele 12-GGC compared to adjacent normal tissues. In addition, Miri et al. conducted a study on the link between the GSPT1 gene in breast cancer patients and the susceptibility to breast cancer. The study showed that there were significantly elevated GSPT1 expression levels in breast cancer tissues, and the presence of the longer allele 12-GGC in the GSPT1 exon can increase the risk of developing breast cancer threefold. Therefore, some scholars believe that the link between the allele 12-GGC and cancer progression is achieved by GSPT1 regulating mRNA degradation, translation efficiency, or the like to make proteins undergo functional loss.

In developing countries, gastric cancer is a leading cause of cancer-related deaths. Malta-Vacas et al. found that the expression level of GSPT1 in intestinal-type gastric tumors was significantly higher than that in diffuse gastric tumors. In addition, they evaluated the link between GSPT1 and the potential genetic susceptibility to gastric cancer, and found that patients with the allele 12-GGC had a 20-fold increased risk of developing gastric cancer compared to normal people, regardless of genotype. Tian et al. explored the potential influence of GSPT1 on the development of gastric cancer and found that the expression level of GSPT1 was significantly elevated in gastric cancer tissues. Some scholars believe that the overexpression of GSPT1 promotes tumor progression by enhancing the translation efficiency of particular oncogene mRNAs, while some attribute it to other roles of GSPT1, such as regulating the cell cycle, cell apoptosis, etc. Since GSPT1 is also involved in cytoskeleton formation and controls chromosomal segregation, the influence of GSPT1 on chromosomal segregation and cytokinesis may be a potential mechanism through which this gene influences the development of gastric cancer.

Liver cancer is a common neoplastic disease. Clinical data indicate that the expression level of GSPT1 is generally higher in liver cancer tissues than in normal cells. Since one of the main functions of GSPT1 is to regulate the transition of cells from the G1 phase to the S phase, some researchers speculate that interfering with GSPT1 mRNA expression may alter the cell cycle, leading to an increase in the percentage of cells in the G1 phase. This, in turn, reduces the viability and proliferative capacity of tumor cells. Although GSPT1 has a cancer-promoting effect in the development and progression of liver cancer, researchers found, by constructing HepG2 cells with overexpression and knockdown of the GSTP1 gene, that GSPT1 may inhibit canceration in the progression of liver cancer.

Colorectal cancer is one of the most common malignancies in the digestive system. A study by Xiao et al. demonstrated that GSPT18 was overexpressed in human colorectal cancer HCT116 cells compared to colorectal cells in the normal control group, and further experiments showed that knocking down GSPT1 in colorectal cancer cells with high GSPT1 expression could inhibit the proliferation and migration of colorectal cancer cells. Research findings show that GSPT1 inhibits cell cycle progression in HCT116 cells via the mTOR pathway. When the mTOR pathway is activated, the translation of a subset of mRNAs is increased. Therefore, GSPT1 may play the role of a proto-oncogene in the development and progression of colorectal cancer.

In summary, GSPT1 plays an important role in the development and progression of related solid tumors. However, due to its functional diversity and complex regulatory mechanisms, it plays different roles in different tumors. Currently, it is widely recognized that GSPT1 is a proto-oncogene of most tumors and, in certain particular types of tumors, plays the role of a tumor suppressor gene. Although there remains a lack of relatively in-depth research on the related mechanisms of action of GSPT1 in tumors, there is no doubt that GSPT1 has become a powerful potential target in tumor treatment.

### SUMMARY

Objectives: One of the objectives of the present disclosure is to provide a phthalazinone compound of general formula I or a pharmaceutically acceptable salt, tautomer, mesomer, racemate, enantiomer, or diastereomer thereof: 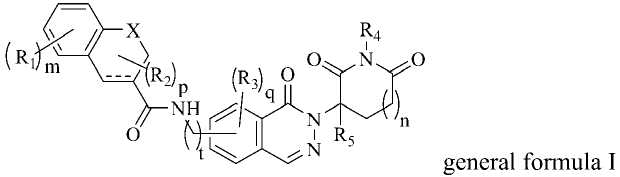 wherein:
- - - - represents a single or double bond;
X is selected from -CH₂-, -NH-, -O-, -S-, and -Se-;
each R₁ is independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, nitro, - C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, or heteroaryl is unsubstituted or optionally substituted with 1-3 Rₐ groups;
each R₂ is independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, nitro, - C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, or heteroaryl is unsubstituted or optionally substituted with 1-3 Rₐ groups;
each R₃ is independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, nitro, - C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, or heteroaryl is unsubstituted or optionally substituted with 1-3 Rₐ groups;
R₄ is selected from hydrogen, deuterium, C₁-C₁₂ alkyl, 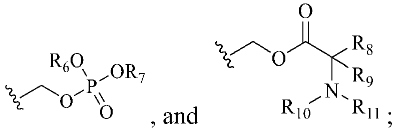
R₅ is selected from hydrogen, deuterium, and C₁-C₁₂ alkyl;
R₆, R₇, R₈, R₉, R₁₀, and R₁₁ are each independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, nitro, -C(O)NRₐR_{b}, -C(O)Rₐ, -C(O)ORₐ, -ORₐ, -OC(O)Rₐ, -OC(O)ORₐ, - OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, or heteroaryl is unsubstituted or optionally substituted with 1-3 R ₐ groups; Rₐ and R_{b} are each independently selected from hydrogen, C₁-C₆ alkyl, C₂-C₆ alkenyl, C₂-C₆ alkynyl, halogen, hydroxyl, cyano, nitro, benzyl, -C(O)NR_{c}R_{d}, -C(O)R_{c}, -C(O)OR_{c}, -OR_{c}, - OC(O)R_{c}, -OC(O)OR_{c}, -OC(O)NR_{c}R_{d}, -NR_{c}R_{d}, -SR_{c}, -S(O)R_{c}, -S(O)₂R_{c}, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, heteroaryl, or benzyl is unsubstituted or optionally substituted with 1-3 R_{c} groups;
R_{c} and R_{d} are each independently selected from hydrogen, halogen, carbonyl, hydroxyl, cyano, nitro, phenyl, benzyl, C₁-C₆ alkyl, halogenated C₁-C₆ alkyl, C₁-C₆ alkoxy, halogenated C₁-C₆ alkoxy, C₃-C₆ cycloalkyl, and halogenated C₃-C₆ cycloalkyl;
m, p, q, t, and n are each independently selected from 0, 1, 2, and 3.
In certain preferred embodiments,
X is selected from -NH- and -0-.

In certain preferred embodiments,
each R₁ is independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, hydroxyl, nitro, C₁-C₁₀ aryl, and -NR_{aRb};
Rₐ and R_{b} are each independently selected from hydrogen, C₁-C₆ alkyl, and -C(O)R_{c};
R_{c} is selected from hydrogen, halogen, C₁-C₆ alkyl, halogenated C₁-C₆ alkyl, C₁-C₆ alkoxy, halogenated C₁-C₆ alkoxy, and C₃-C₆ cycloalkyl.

In certain more preferred embodiments,
each R₁ is independently selected from hydrogen, methyl, fluorine, chlorine, bromine, methoxy, trifluoromethyl, trifluoromethoxy, vinyl, tert-butyl, and phenyl.

In certain preferred embodiments,
R₂ and R₃ are each independently selected from hydrogen.

In certain preferred embodiments,
R₄ and R₅ are each independently selected from hydrogen.

In certain preferred embodiments,
p, q, and n are each independently selected from 1.

In certain preferred embodiments,
m is selected from 0, 1, and 2.

In certain preferred embodiments,
t is selected from 0 and 1.

The above compound of general formula I to which the present disclosure relates may also be present in the form of salts thereof, which are converted *in vivo* into the compound of general formula 1. For example, within the scope of the present disclosure, according to a process known in the art, the compounds of the present disclosure are converted into pharmaceutically acceptable salt forms and used in the salt forms.

In some preferred embodiments, the pharmaceutically acceptable salt includes, but is not limited to, acid addition salts formed by the compound of general formula I with the following acids: hydrochloric acid, hydrobromic acid, sulfuric acid, phosphoric acid, methanesulfonic acid, benzenesulfonic acid, p-toluenesulfonic acid, naphthalenesulfonic acid, citric acid, tartaric acid, lactic acid, pyruvic acid, acetic acid, maleic acid or succinic acid, fumaric acid, salicylic acid, phenylacetic acid, and mandelic acid; and acid salts formed by the compound of general formula I with inorganic bases.

In some more preferred embodiments, the pharmaceutically acceptable salt includes, but is not limited to, alkali metal cation salts, alkaline earth metal cation salts, and ammonium cation salts. The compound of general formula **I** of the present disclosure is preferably the following compounds:

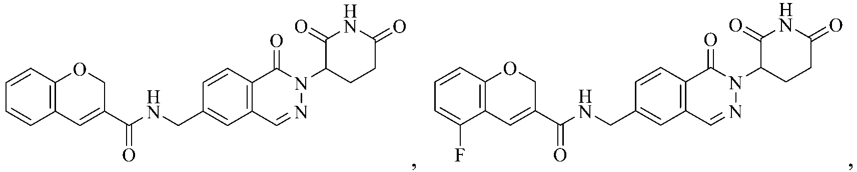

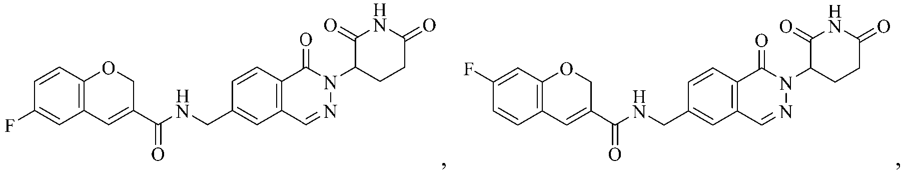

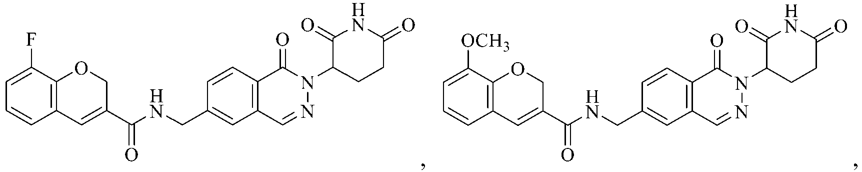

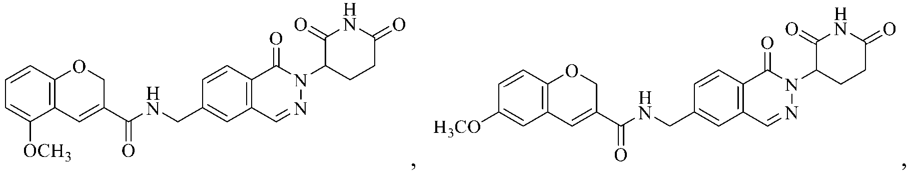

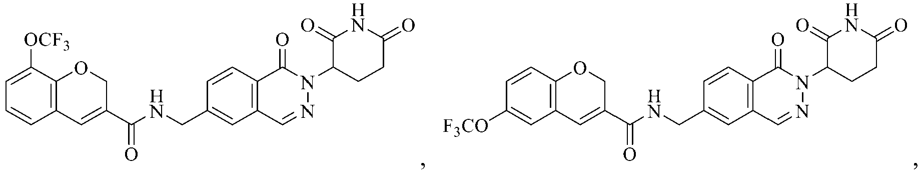

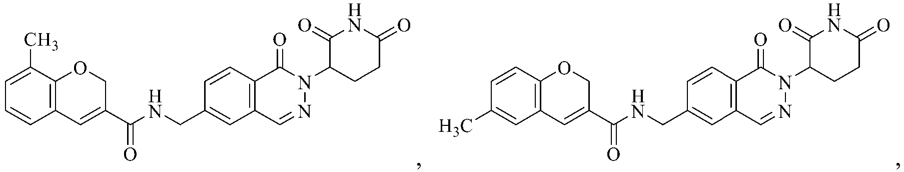

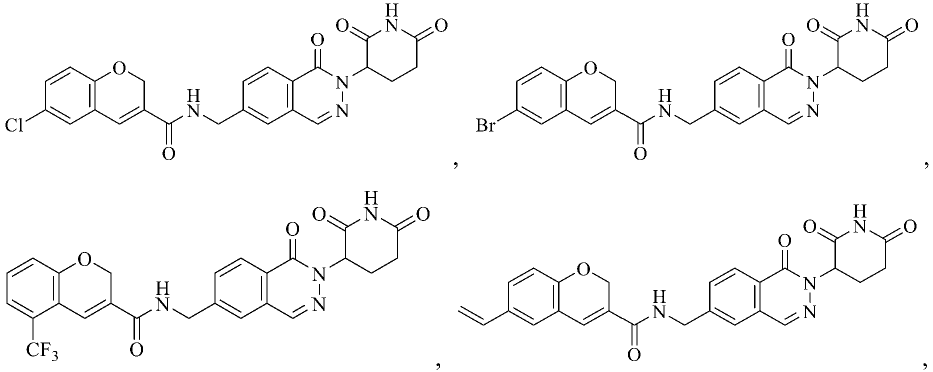

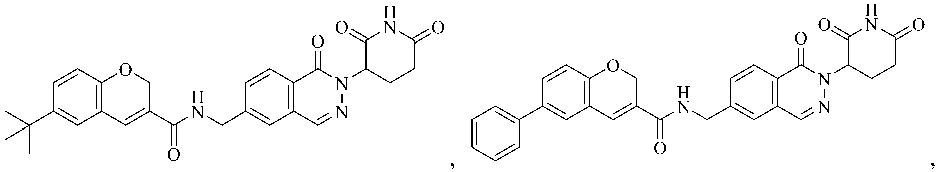

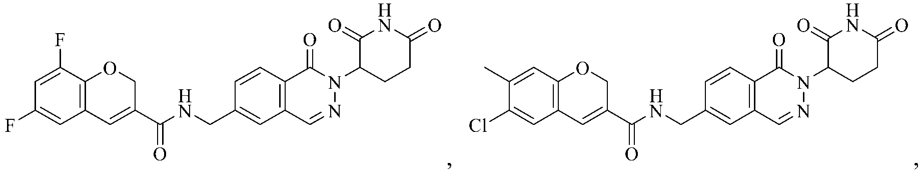

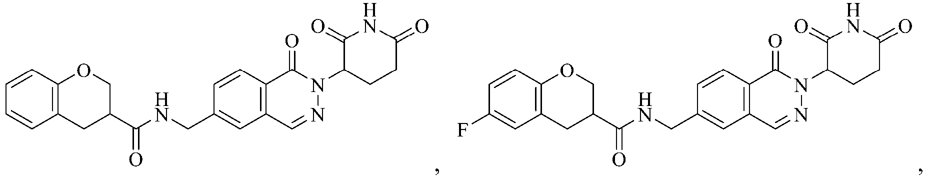

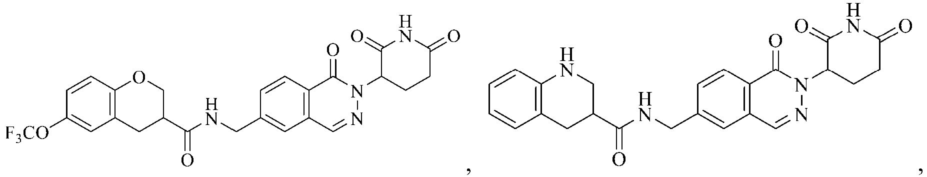

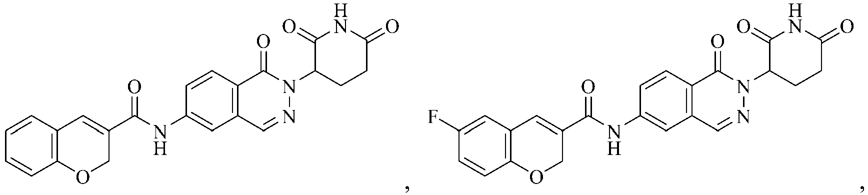

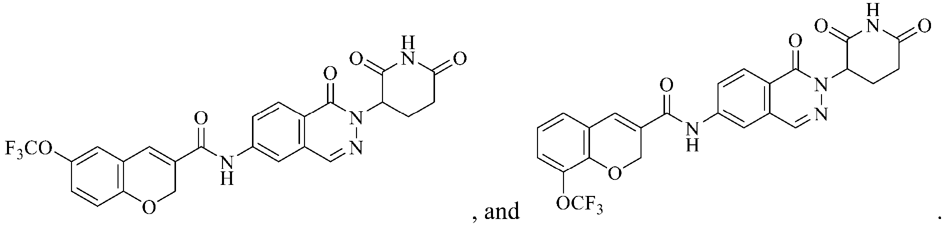

Another objective of the present disclosure is to provide a pharmaceutical composition comprising a therapeutically effective amount of the compound of general formula I or the pharmaceutically acceptable salt, tautomer, mesomer, racemate, enantiomer, or diastereomer thereof and a pharmaceutically acceptable carrier or excipient.

The pharmaceutical composition of the present disclosure can be administered in various known ways, e.g., orally, parenterally, by inhalation spray, or via an implanted reservoir. The pharmaceutical composition of the present disclosure can be administered alone or in combination with other drugs. A composition for oral administration may be any orally acceptable dosage form, including but not limited to tablets, capsules, emulsions and suspensions, dispersions, and solutions. Commonly used pharmaceutically acceptable carriers or excipients include stabilizers, diluents, surfactants, lubricants, antioxidants, binders, colorants, fillers, emulsifiers, etc.

A sterile injectable composition can be formulated according to techniques known in the art using suitable dispersing or wetting agents and suspending agents. Pharmaceutically acceptable carriers and solvents that can be used include water, mannitol, sodium chloride solution, etc. The actual dosage level of the active ingredient in the pharmaceutical composition of the present disclosure may be varied so as to obtain an amount of the active ingredient that is effective to achieve the desired therapeutic response for a particular patient, composition, and mode of administration, without being toxic to the patient. The selected dosage level will depend upon a variety of factors, including the activity of the specific compound or salt thereof of the present disclosure employed, the route of administration, the time of administration, the rate of excretion of the specific composition employed, the duration of the treatment, other drugs, compounds, and/or materials used in combination with the specific composition employed, the age, sex, body weight, general health, and prior medical history of the patient being treated, and similar factors well known in the medical arts.

Another objective of the present disclosure is to provide use of the compound of general formula I or the pharmaceutically acceptable salt, tautomer, mesomer, racemate, enantiomer, or diastereomer thereof in the preparation of a medicament for treating or preventing a disease related to mutation, unbalanced expression, allosterism, and dysfunction of GSPT1, IKZF1, IKZF2, IKZF3, CK1α, N-MYC, or C-MYC protein.

The related disease is cancer, a viral infection, aging, an immune disease, or a neurological disease, wherein the cancer is selected from acute myeloid leukemia, liver cancer, acute lymphocytic leukemia, bladder cancer, bone cancer, brain cancer, breast cancer, cervical cancer, choriocarcinoma, chronic lymphocytic leukemia (CLL), chronic granulocytic leukemia (CML), colon cancer, esophageal cancer, gallbladder cancer, gastric cancer, gastrointestinal stromal tumor, head and neck cancer, Hodgkin lymphoma, laryngeal cancer, lung cancer, melanoma, mesothelioma, multiple myeloma, ovarian cancer, pancreatic cancer, prostate cancer, rectal cancer, renal cancer, skin cancer, testicular cancer, throat cancer, thyroid cancer, and uterine cancer. The lung cancer is non-small cell lung cancer.

The present disclosure further provides use of the compound of general formula I or the pharmaceutically acceptable salt, tautomer, mesomer, racemate, enantiomer, or diastereomer thereof in the preparation of a GSPT1 degrader.

Beneficial Effects:
The phthalazinone compounds of the present disclosure exhibit a highly significant degradation effect on the GSPT1 protein and antiproliferative inhibitory activity against AML cells.

Therefore, the phthalazinone compounds of the present disclosure can be used to prepare a medicament for treating or preventing a disease related to mutation, unbalanced expression, allosterism, and dysfunction of GSPT1, IKZF1, IKZF2, IKZF3, CK1α, N-MYC, or C-MYC protein; in addition, the compounds can be used to prepare a GSPT1 degrader.

Unless otherwise stated, the terms used in the specification and claims have the following meanings.

The term "isomer" includes enantiomeric, diastereomeric, and geometric (or conformational) isomeric forms of a given structure. For example, the present application includes R and S configurations for each asymmetric center, Z and E double bond isomers, Z and E conformational isomers, single stereochemical isomers, and enantiomeric, diastereomeric, and geometric (or conformational) isomer mixtures.

The term "pharmaceutically acceptable salt" refers to, for example, an acid addition salt thereof and/or a base salt thereof. Suitable acid addition salts are formed from acids that form non-toxic salts, e.g., hydrochlorides/chlorides. Suitable base salts are formed from bases that form non-toxic salts, e.g., calcium salts and sodium salts. Hemisalts of acids and bases, e.g., hemisulphates and hemicalcium salts, may also be formed.

The term "therapeutically effective amount" refers to an amount of a compound of the present application that (i) treats a specific disease, condition, or disorder; (ii) palliates, alleviates, or eliminates one or more symptoms of a specific disease, condition, or disorder; or (iii) prevents or delays the onset of one or more symptoms of the specific disease, condition, or disorder described in the present application.

The term "pharmaceutically acceptable carrier or excipient" refers to a non-toxic carrier, auxiliary material, or vehicle that does not destroy the pharmacological activity of the compound with which it is formulated.

The term "alkyl" refers to a saturated aliphatic hydrocarbon group. Alkyl groups containing 1 to 6 carbon atoms are preferred in the present disclosure. Non-limiting examples of lower alkyl groups containing 1 to 6 carbon atoms include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc.

The term "alkenyl" refers to an aliphatic hydrocarbon having at least one carbon-carbon double bond, including linear and branched chains having at least one carbon-carbon double bond. The alkenyl groups of the present disclosure preferably have 2 to 6 carbon atoms. For example, the term "C₂₋₆ alkenyl" includes linear or branched unsaturated groups of 2 to 6 carbon atoms (having at least one carbon-carbon double bond), including but not limited to vinyl, 1-propenyl, 2-propenyl (allyl), isopropenyl, 2-methyl-1-propenyl, 1-butenyl, 2-butenyl, etc.

The term "alkynyl" refers to an aliphatic hydrocarbon having at least one carbon-carbon triple bond, including linear and branched chains having at least one carbon-carbon triple bond. The alkynyl groups of the present disclosure have 2 to 6 carbon atoms. For example, "C₂₋₆ alkynyl" includes linear or branched hydrocarbon chain alkynyl groups as defined above having 2 to 6 carbon atoms.

The term "alkoxy" refers to -O-(alkyl) and -O-(unsubstituted cycloalkyl), wherein the alkyl is as defined above. Non-limiting examples of alkoxy groups include methoxy, ethoxy, propoxy, butoxy, cyclopropyloxy, cyclobutoxy, cyclopentyloxy, and cyclohexyloxy.

The term "cycloalkyl" refers to a saturated or partially unsaturated monocyclic or polycyclic hydrocarbon substituent. The cycloalkyl groups of the present disclosure preferably contain 3 to 10 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl groups include spiro-ring, fused-ring, and bridged-ring cycloalkyl groups.

The term "spirocycloalkyl" refers to a 5- to 20-membered polycyclic group in which one carbon atom (referred to as a spiro atom) is shared between monocyclic rings; it may contain one or more double bonds, but none of the rings has a fully conjugated π-electron system. It is preferably 6- to 14-membered, and is more preferably 7- to 10-membered (e.g., 7-, 8-, 9-, or 10-membered). According to the number of spiro atoms shared between the rings, spirocycloalkyl may be monospirocycloalkyl, bispirocycloalkyl, or polyspirocycloalkyl, preferably monospirocycloalkyl and bispirocycloalkyl, and more preferably 4-membered/4-membered, 4-membered/5-membered, 4-membered/6-membered, 5-membered/5-membered, or 5-membered/6-membered monospirocycloalkyl.

The term "fused cycloalkyl" refers to a 5- to 20-membered all-carbon polycyclic group in which each ring in the system shares a pair of adjacent carbon atoms with the other rings in the system, wherein one or more rings may contain one or more double bonds, but none of the rings has a fully conjugated π-electron system. It is preferably 6- to 14-membered, and is more preferably 7- to 10-membered. According to the number of constituent rings, it may be bicyclic, tricyclic, tetracyclic, or polycyclic fused cycloalkyl, preferably bicyclic or tricyclic, and more preferably 5-membered/5-membered or 5-membered/6-membered bicycloalkyl.

The term "bridged cycloalkyl" refers to a 5- to 20-membered all-carbon polycyclic group in which any two rings share two carbon atoms that are not directly connected; it may contain one or more double bonds, but none of the rings has a fully conjugated π-electron system. It is preferably 6- to 14-membered, and is more preferably 7- to 10-membered. According to the number of constituent rings, it may be bicyclic, tricyclic, tetracyclic, or polycyclic bridged cycloalkyl, preferably bicyclic, tricyclic, or tetracyclic, and more preferably bicyclic or tricyclic. The term "heterocyclyl" refers to a saturated or partially unsaturated monocyclic or polycyclic hydrocarbon substituent containing 3 to 20 ring atoms, wherein one or more of the ring atoms are heteroatoms selected from nitrogen, oxygen, and S(O)m (where m is an integer from 0 to 2), but a cyclic portion of -O-O-, -O-S-, or -S-S- is excluded, and the other ring atoms are carbon atoms. Preferably, it contains 3 to 10 ring atoms, of which 1-4 are heteroatoms; most preferably, it contains 3 to 8 ring atoms, of which 1-3 are heteroatoms; most preferably, it contains 5 to 6 ring atoms, of which 1-2 or 1-3 are heteroatoms. Non-limiting examples of monocyclic heterocyclyl groups include pyrrolidinyl, imidazolidinyl, tetrahydrofuranyl, tetrahydropyranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, etc., preferably tetrahydropyranyl, piperidinyl, and pyrrolidinyl. Polycyclic heterocyclyl groups include spiroheterocyclyl groups, fused heterocyclyl groups, and bridged heterocyclyl groups.

The term "spiroheterocyclyl" refers to a 5- to 20-membered polycyclic heterocyclyl group in which one atom (referred to as a spiro atom) is shared between monocyclic rings, wherein one or more ring atoms are heteroatoms selected from nitrogen, oxygen, and S(O)m (where m is an integer from 0 to 2), and the other ring atoms are carbon atoms. It may contain one or more double bonds, but none of the rings has a fully conjugated π-electron system. It is preferably 6-to 14-membered, and is more preferably 7- to 10-membered. According to the number of spiro atoms shared between the rings, spiroheterocyclyl may be monospiroheterocyclyl, bispiroheterocyclyl, or polyspiroheterocyclyl, preferably monospiroheterocyclyl and bispiroheterocyclyl, and more preferably 4-membered/4-membered, 4-membered/5-membered, 4-membered/6-membered, 5-membered/5-membered, or 5-membered/6-membered monospiroheterocyclyl.

The term "fused heterocyclyl" refers to a 5- to 20-membered polycyclic heterocyclyl group in which each ring in the system shares a pair of adjacent atoms with the other rings in the system, wherein one or more rings may contain one or more double bonds, but none of the rings has a fully conjugated π-electron system; one or more ring atoms are heteroatoms selected from nitrogen, oxygen, and S(O)m (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic fused heterocyclyl, preferably bicyclic or tricyclic, and more preferably 5-membered/5-membered or 5-membered/6-membered bicyclic fused heterocyclyl.

The term "bridged heterocyclyl" refers to a 5- to 14-membered polycyclic heterocyclyl group in which any two rings share two atoms that are not directly connected; it may contain one or more double bonds, but none of the rings has a fully conjugated π-electron system, wherein one or more ring atoms are heteroatoms selected from nitrogen, oxygen, and S(O)m (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic bridged heterocyclyl, preferably bicyclic, tricyclic, or tetracyclic, and more preferably bicyclic or tricyclic.

The heterocyclyl includes those in which the heterocyclyl described above (including monocyclic, spiroheterocyclic, fused heterocyclic, and bridged heterocyclic rings) is fused to an aryl, heteroaryl, or cycloalkyl ring, wherein the ring attached to the parent structure is heterocyclyl; its non-limiting examples include:

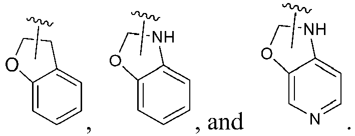

The term "aryl" refers to a 6- to 14-membered, preferably 6- to 10-membered, all-carbon monocyclic or fused polycyclic (i.e., rings sharing a pair of adjacent carbon atoms) group having a conjugated π-electron system, such as phenyl and naphthyl.

The term "heteroaryl" refers to a heteroaromatic system containing 1 to 4 heteroatoms and 5 to 14 ring atoms, wherein the heteroatoms are selected from oxygen, sulfur, and nitrogen. Preferably, heteroaryl groups are 5- to 10-membered and contain 1 to 3 heteroatoms; more preferably, heteroaryl groups are 5- or 6-membered and contain 1 to 2 heteroatoms; preferably, heteroaryl groups are, for example, imidazolyl, furanyl, thienyl, thiazolyl, pyrazolyl, oxazolyl, pyrrolyl, tetrazolyl, pyridinyl, pyrimidinyl, thiadiazole, pyrazinyl, pyridazinyl, etc.

The heteroaryl includes those in which the heteroaryl described above is fused to an aryl, heterocyclyl, or cycloalkyl ring, wherein the ring attached to the parent structure is a heteroaryl ring; its non-limiting examples include:

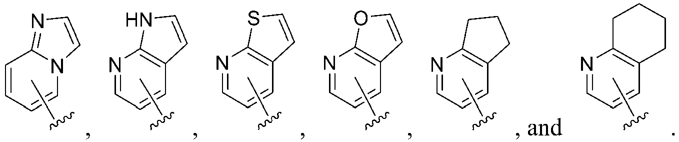

The term "hydroxyalkyl" refers to an alkyl group substituted with a hydroxyl group, wherein the alkyl is as defined above.

The term "haloalkyl" refers to an alkyl group substituted with one or more halogens, wherein the alkyl is as defined above.

The term "haloalkoxy" refers to an alkoxy group substituted with one or more halogens, wherein the alkoxy is as defined above.

### DETAILED DESCRIPTION

The technical solutions of the present disclosure are described in detail below using specific examples, but the scope of protection of the present disclosure is not limited to the examples.

### Synthesis of key intermediate-1:

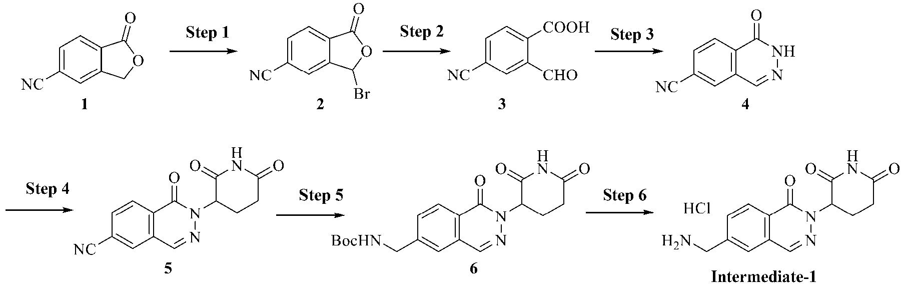

Step 1: 5-Cyanophthalide (25.00 g, 157.20 mmol) was dissolved in 400 mL of carbon tetrachloride, and N-bromosuccinimide (49.00 g, 282.96 mmol) and azobisisobutyronitrile (13.00 g, 78.60 mmol) were added sequentially. The mixture was heated to reflux and stirred for 10 h. The mixture was cooled to room temperature and then filtered under vacuum. The filtrate was concentrated, and the residue was purified by silica gel column chromatography to give compound **2** as a white solid (21.57 g, yield: 57.90%). HRMS (ESI⁺):(M + H)⁺ found 237.9486. Step 2: 3-Bromo-1-oxo-1,3-dihydroisobenzofuran-5-carbonitrile (21.57 g, 91.02 mmol) was dissolved in 300 mL of water, and the mixture was heated to reflux and stirred for 4 h. The mixture was cooled to room temperature and then filtered under vacuum, and the filter cake was dried to give compound **3** as a white solid (12.16 g, yield: 76.3%). HRMS (ESI⁻):(M + H)⁺ found 174.0188.

Step 3: 4-Cyano-2-formylbenzoic acid (12.16 g, 69.44 mmol) was dissolved in 50 mL of 95% ethanol. 80% hydrazine hydrate (13.90 g, 347.24 mmol) was added dropwise, and the mixture was heated to 80 °C and stirred for 30 min. The mixture was cooled to room temperature and then filtered under vacuum, and drying was performed to give compound **4** as a brownish-yellow solid (10.01 g, yield: 84.3%). HRMS (ESI⁺):(M + H)⁺ found 172.0510.

Step 4: 1-Oxo-1,2-dihydrophthalazine-6-carbonitrile (10.01 g, 58.52 mmol) was dissolved in 50 mL of N,N-dimethylformamide. Sodium hydride (2.80 g, 117.07 mmol) was added in an ice bath, and the mixture was stirred at room temperature for 1 h. 3-Bromopiperidine-2,6-dione (22.35 g, 117.07 mmol) was added, and the mixture was heated to 100 °C and stirred for 48 h. The mixture was cooled to room temperature, and the reaction liquid was poured into water. The mixture was extracted with ethyl acetate. The organic phases were combined, washed sequentially with water and saturated brine, dried, and concentrated. The residue was purified by silica gel column chromatography to give compound **5** as a white solid (6.81 g, yield: 41.3%). HRMS (ESI⁺):(M + H)⁺ found 283.0820.

Step 5: 2-(2,6-Dioxopiperidin-3-yl)-1-oxo-1,2-dihydrophthalazine-6-carbonitrile (6.81 g, 24.17 mmol) was dissolved in 30 mL of N,N-dimethylformamide. Di-tert-butyl dicarbonate (10.54 g, 48.34 mmol) and 15 mL of Raney nickel were added sequentially. The mixture was fully purged with hydrogen, heated to 50 °C, and stirred for 5 h. The mixture was filtered under vacuum, and the filtrate was poured into water. The mixture was extracted with ethyl acetate. The organic phases were combined, washed sequentially with water and saturated brine, dried, and concentrated to give compound **6** as a yellow solid (6.14 g, yield: 65.8%). HRMS (ESI⁺):(M + H)⁺ found 387.1665.

Step 6: tert-Butyl ((2-(2,6-dioxopiperidin-3-yl)-1-oxo-1,2-dihydrophthalazin-6-yl)methyl)carbamate (6.14 g, 15.90 mmol) was dissolved in 30 mL of a solution of hydrochloric acid in dioxane (4 M), and the solution was stirred at room temperature for 3 h. After filtration under vacuum, the filter cake was dried to give a white solid, which was **intermediate-1:** 3-(6-(aminomethyl)-1-oxophthalazin-2(1H)-yl)piperidine-2,6-dione hydrochloride (3.71 g, yield: 72.4%). HRMS (ESI⁺):(M + H)⁺ found 287.1130.

### Synthesis of key intermediate-2:

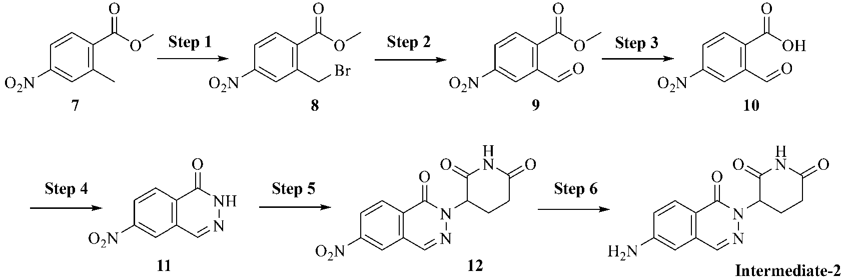

Step 1: Methyl 2-methyl-4-nitrobenzoate (10.00 g, 51.24 mmol) was dissolved in 200 mL of 1,2-dichloroethane, N-bromosuccinimide (13.68 g, 76.86 mmol) and benzoyl peroxide (3.26 g, 13.48 mmol) were added sequentially, and the mixture was heated to reflux and stirred for 3 h. The mixture was cooled to room temperature and then concentrated, and the residue was purified by silica gel column chromatography to give compound **8** as a white solid (8.71 g, yield: 62.0%). HRMS (ESI⁺):(M + H)⁺ found 273.9711.

Step 2: Methyl 2-bromomethyl-4-nitrobenzoate (8.71 g, 31.78 mmol) was dissolved in 300 mL of dichloromethane, N-methylmorpholine oxide (8.55 g, 73.00 mmol) and a small number of molecular sieves (4A) were added sequentially, and the mixture was stirred at room temperature for 4 h. After filtration under vacuum, the filtrate was concentrated, and the residue was purified by silica gel column chromatography to give compound **9** as a white solid (4.67 g, yield: 70.2%). HRMS (ESI⁺): found 210.0388.

Step 3: Methyl 2-formyl-4-nitrobenzoate (4.67 g, 22.33 mmol) was dissolved in 25 mL of tetrahydrofuran, a 5 M aqueous lithium hydroxide solution (25 mL) was added, and the mixture was stirred at room temperature for 2 h. The organic solvent was removed by concentration, the pH was adjusted to 2-3 with 1 N dilute hydrochloric acid at 0 °C, and the mixture was filtered under vacuum. The filter cake was washed with water and then dried to give compound **10** as a white solid (4.00 g, yield: 91.9%). HRMS (ESI⁻):(M - H)⁻ found 194.0087.

Step 4: 2-Formyl-4-nitrobenzoic acid (4.00 g, 20.50 mmol) was dissolved in 40 mL of ethanol, 80% hydrazine hydrate (6.41 g, 102.5 mmol) was added, and the mixture was stirred at 80 °C for 2 h. The mixture was cooled to room temperature and then filtered under vacuum, and the filter cake was washed with a small amount of ethanol and then dried to give compound **11** as a yellow solid (3.37 g, yield: 85.9%). HRMS (ESI⁺):(M + H)⁺ found 192.0401.

Step 5: 6-Nitrophthalazin-1(2H)-one (3.37 g, 17.63 mmol) was dissolved in 150 mL of N,N-dimethylformamide, sodium hydride (1.41 g, 35.26 mmol) was added, and the mixture was stirred at room temperature under nitrogen atmosphere for 1 h. 3-Bromopiperidine-2,6-dione (5.08 g, 26.45 mmol) was added, and the mixture was stirred at 85 °C for 12 h. The mixture was cooled to room temperature and filtered under vacuum, and the filtrate was poured into water. The mixture was extracted with ethyl acetate. The organic layers were combined, washed sequentially with water and saturated brine, dried, and concentrated. The residue was purified by silica gel column chromatography to give compound **12** as a white solid (2.14 g, yield: 40.2%). HRMS (ESI⁺):(M + H)⁺ found 303.0711.

Step 6: 3-(6-Nitro-1-oxophthalazin-2(1H)-yl)piperidine-2,6-dione (2.14 g, 7.08 mmol) was dissolved in 50 mL of methanol, and 10% Pd/C (1.00 g) was added. After the addition, the mixture was stirred at room temperature under hydrogen atmosphere for 8 h. The mixture was filtered under vacuum, and the filtrate was concentrated under vacuum to give a white solid, which was **intermediate-2:** 3-(6-amino-1-oxophthalazin-2(1H)-yl)piperidine-2,6-dione (1.55 g, yield: 80.2%). HRMS (ESI⁺):(M + H)⁺ found 273.0972.

### Synthesis of key intermediate-3:

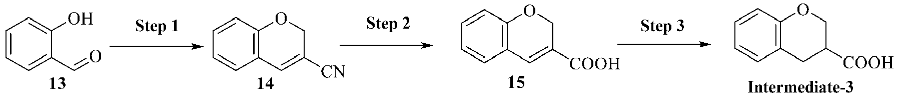

Step 1: 2-Hydroxybenzaldehyde (2.00 g, 16.39 mmol) was dissolved in 15 mL of acrylonitrile, and 1,4-diazabicyclo[2.2.2]octane (1.84 g, 16.39 mmol) was added. After the addition, the mixture was heated to reflux and stirred for 3 h under nitrogen atmosphere. The reaction liquid was poured into 30 mL of a 10% aqueous sodium hydroxide solution, and the mixture was extracted with ethyl acetate. The organic phases were combined, washed with saturated brine, dried, and concentrated to give compound **14** as a yellow crystalline solid (2.30 g, yield: 90.2%). HRMS (ESI⁺):(M + H)⁺ found 158.0621.

Step 2: 2H-Chromene-3-carbonitrile (2.30 g, 14.73 mmol) was dissolved in 40 mL of a 10% aqueous sodium hydroxide solution, and the mixture was heated to 60 °C and stirred for 10 h. Dilute hydrochloric acid (2 M) was added to adjust the pH to 4-5. The mixture was filtered under vacuum and dried to give compound **15** as a yellow solid (1.65 g, yield: 63.7%). HRMS (ESI⁻):(M - H)⁻ found 175.0400.

Step 3: 2H-Chromene-3-carboxylic acid (1.50 g, 8.52 mmol) was dissolved in 20 mL of methanol, and 10% Pd/C (1.00 g) was added. The mixture was stirred at room temperature for 8 h. The mixture was filtered under vacuum, and the filtrate was concentrated under vacuum to give a brownish-yellow solid, which was **intermediate-3:** chroman-3-carboxylic acid (0.76 g, yield: 50.2%). HRMS (ESI⁻):(M - H)⁻ found 177.0545.

### Example 1: N-((2-(2,6-Dioxopiperidin-3-yl)-1-oxo-1,2-dihydrophthalazin-6-yl)methyl)-2H-chromene-3-carboxamide (Compound T-1)

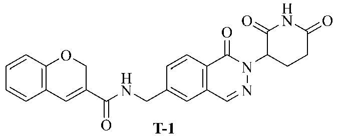

Intermediate-1 (0.10 g, 0.31 mmol) was dissolved in 5 mL of N,N-dimethylformamide, and compound 15 (0.07 g, 0.38 mmol) and HATU (0.18 g, 0.47 mmol) were added sequentially. The mixture was stirred at room temperature for 1 h. DIPEA (0.12 g, 0.94 mmol) was added, and the mixture was stirred at room temperature overnight. The reaction liquid was poured into ice water, and the mixture was filtered under vacuum. The filter cake was purified by silica gel column chromatography to give a white solid, which was compound T-1 (0.10 g, yield: 72.6%, purity: 98.93%). ¹H NMR (300 MHz, DMSO-*d*₆) *δ* (ppm) 11.06 (s, 1H), 9.02 (s, 1H), 8.55 (s, 1H), 8.20 (d, *J* = 6.4 Hz, 1H), 7.84 (s, 1H), 7.39 (s, 1H), 7.12-7.06 (m, 2H), 6.93 (t, *J* = 8.2 Hz, 1H), 6.92-6.87 (m, 2H), 5.95-5.83 (m, 1H), 5.15 (d, *J* = 6.2 Hz, 2H), 4.64 (s, 2H), 2.97-2.85 (m, 1H), 2.64 (d, *J* = 7.9 Hz, 2H), 2.12 (s, 1H).

### Example 2: N-((2-(2,6-Dioxopiperidin-3-yl)-1-oxo-1,2-dihydrophthalazin-6-yl)methyl)-5-fluoro-2H-chromene-3-carboxamide (Compound T-2)

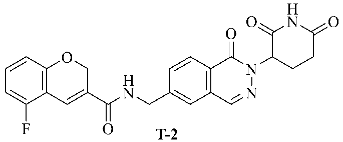

Synthesis of 5-fluoro-2H-chromene-3-carboxylic acid: With reference to the synthesis method for intermediate-3, 6-fluorosalicylaldehyde was used as the starting material to prepare the compound, with a two-step yield of 52.4%. HRMS (ESI⁻):(M - H)⁻ found 193.0318.

Synthesis of compound T-2: With reference to the synthesis method for compound T-1, 5-fluoro-2H-chromene-3-carboxylic acid was used as the starting material to prepare compound T-2 (a white solid, 0.08 g, yield: 55.8%, purity: 96.93%). ¹H NMR (300 MHz, DMSO-*d*₆) *δ* (ppm) 11.06 (s, 1H), 9.05 (s, 1H), 8.52 (s, 1H), 8.25 (d, *J* = 7.4 Hz, 1H), 7.84 (d, *J* = 9.5 Hz, 2H), 7.32 (s, 1H), 7.16-7.06 (m, 2H), 6.91-6.87 (m, 1H), 5.85-5.79 (m, 1H), 5.05 (s, 2H), 4.61 (d, *J* = 5.2 Hz, 2H), 2.99-2.87 (m, 1H), 2.64 (d, *J* = 16.1 Hz, 2H), 2.12 (s, 1H).

### Example 3: N-((2-(2,6-Dioxopiperidin-3-yl)-1-oxo-1,2-dihydrophthalazin-6-yl)methyl)-6-fluoro-2H-chromene-3-carboxamide (Compound T-3)

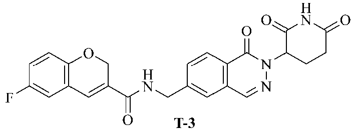

Synthesis of 6-fluoro-2H-chromene-3-carboxylic acid: With reference to the synthesis method for intermediate-3, 5-fluorosalicylaldehyde was used as the starting material to prepare the compound, with a two-step yield of 62.1%. HRMS (ESI⁻):(M - H)⁻ found 193.0310.

Synthesis of compound T-3: With reference to the synthesis method for compound T-1, 6-fluoro-2H-chromene-3-carboxylic acid was used as the starting material to prepare T-3 (a white solid, 0.12 g, yield: 83.7%, purity: 97.63%). ¹H NMR (300 MHz, DMSO-*d*₆) *δ* (ppm) 11.08 (s, 1H), 9.05 (s, 1H), 8.50 (s, 1H), 8.26 (d, *J* = 7.8 Hz, 1H), 7.84 (d, *J* = 9.5 Hz, 2H), 7.32 (s, 1H), 7.16-7.06 (m, 2H), 6.91-6.87 (m, 1H), 5.85-5.79 (m, 1H), 4.95 (s, 2H), 4.61 (d, *J* = 5.2 Hz, 2H), 2.98-2.89 (m, 1H), 2.64 (d, *J* = 16.1 Hz, 2H), 2.14-2.10 (m, 1H).

### Example 4: N-((2-(2,6-Dioxopiperidin-3-yl)-1-oxo-1,2-dihydrophthalazin-6-yl)methyl)-7-fluoro-2H-chromene-3-carboxamide (Compound T-4)

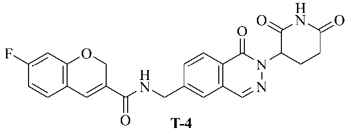

Synthesis of 7-fluoro-2H-chromene-3-carboxylic acid: With reference to the synthesis method for intermediate-3, 4-fluorosalicylaldehyde was used as the starting material to prepare the compound, with a two-step yield of 56.8%. HRMS (ESI⁻):(M - H)⁻ found 193.0321.

Synthesis of compound T-4: With reference to the synthesis method for compound T-1, 7-fluoro-2H-chromene-3-carboxylic acid was used as the starting material to prepare compound T-4 (a white solid, 0.08 g, yield: 55.8%, purity: 96.32%). ¹H NMR (300 MHz, DMSO-*d*₆) *δ* (ppm) 11.09 (s, 1H), 9.01 (s, 1H), 8.52 (s, 1H), 8.27 (d, *J* = 7.2 Hz, 1H), 7.85 (s, 2H), 7.38-7.29 (m, 2H), 6.85 (t, *J* = 11.4 Hz, 2H), 5.87-5.78 (m, 1H), 5.01 (s, 2H), 4.61 (s, 2H), 3.01-2.90 (m, 1H), 2.66 (d, *J* = 16.9 Hz, 2H), 2.15 (s, 1H).

### Example 5: N-((2-(2,6-Dioxopiperidin-3-yl)-1-oxo-1,2-dihydrophthalazin-6-yl)methyl)-8-fluoro-2H-chromene-3-carboxamide (Compound T-5)

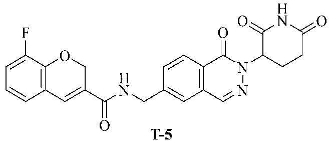

Synthesis of 8-fluoro-2H-chromene-3-carboxylic acid: With reference to the synthesis method for intermediate-3, 3-fluorosalicylaldehyde was used as the starting material to prepare the compound, with a two-step yield of 59.2%. HRMS (ESI⁻):(M - H)⁻ found 193.0311.

Synthesis of compound T-5: With reference to the synthesis method for compound T-1, 8-fluoro-2H-chromene-3-carboxylic acid was used as the starting material to prepare T-5 (a white solid, 0.09 g, yield: 62.8%, purity: 99.83%). ¹H NMR (300 MHz, DMSO-*d*₆) *δ* (ppm) 11.08 (s, 1H), 9.02 (s, 1H), 8.50 (s, 1H), 8.26 (d, *J* = 6.3 Hz, 1H), 7.84 (d, *J* = 9.5 Hz, 2H), 7.30 (s, 1H), 7.16-7.06 (m, 2H), 6.91-6.85 (m, 1H), 5.83-5.76 (m, 1H), 4.97 (s, 2H), 4.61 (d, *J* = 5.2 Hz, 2H), 2.99-2.87 (m, 1H), 2.64 (d, *J* = 16.1 Hz, 2H), 2.14 (s, 1H).

### Example 6: N-((2-(2,6-Dioxopiperidin-3-yl)-1-oxo-1,2-dihydrophthalazin-6-yl)methyl)-8-methoxy-2H-chromene-3-carboxamide (Compound T-6)

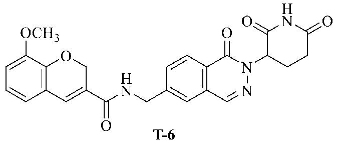

Synthesis of 8-methoxy-2H-chromene-3-carboxylic acid: With reference to the synthesis method for intermediate-3, 2-hydroxy-3-methoxybenzaldehyde was used as the starting material to prepare the compound, with a two-step yield of 56.5%. HRMS (ESI⁻):(M - H)⁻ found 205.0500. Synthesis of compound T-6: With reference to the synthesis method for compound T-1, 8-methoxy-2H-chromene-3-carboxylic acid was used as the starting material to prepare T-6 (a white solid, 0.06 g, yield: 40.8%, purity: 97.63%). ¹H NMR (300 MHz, DMSO-*d*₆) *δ* (ppm) 11.04 (s, 1H), 9.01 (t, *J* = 5.9 Hz, 1H), 8.50 (s, 1H), 8.26 (d, *J* = 8.0 Hz, 1H), 7.84 (d, *J* = 8.7 Hz, 2H), 7.36 (s, 1H), 7.00 (d, *J* = 8.0 Hz, 1H), 6.91 (t, *J* = 8.0 Hz, 1H), 6.84 (d, *J* = 6.3 Hz, 1H), 5.84-5.78 (m, 1H), 4.92 (s, 2H), 4.60 (d, *J* = 5.6 Hz, 2H), 3.76 (s, 3H), 2.99-2.87 (m, 1H), 2.65-2.55 (m, 2H), 2.14-2.09 (m, 1H).

### Example 7: N-((2-(2,6-Dioxopiperidin-3-yl)-1-oxo-1,2-dihydrophthalazin-6-yl)methyl)-5-methoxy-2H-chromene-3-carboxamide (Compound T-7)

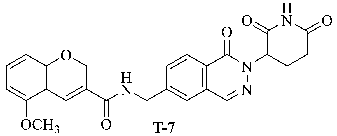

Synthesis of 5-methoxy-2H-chromene-3-carboxylic acid: With reference to the synthesis method for intermediate-3, 6-methoxysalicylaldehyde was used as the starting material to prepare the compound, with a two-step yield of 62.5%. HRMS (ESI⁻):(M - H)⁻ found 205.0515.

Synthesis of compound T-7: With reference to the synthesis method for compound T-1, 5-methoxy-2H-chromene-3-carboxylic acid was used as the starting material to prepare T-7 (a white solid, 0.06 g, yield: 40.8%, purity: 97.85%). ¹H NMR (300 MHz, DMSO-*d*₆) *δ* (ppm) 11.06 (s, 1H), 9.04 (s, 1H), 8.50 (s, 1H), 8.24 (d, *J* = 7.9 Hz, 1H), 7.83 (d, *J* = 8.2 Hz, 2H), 7.53 (s, 1H), 7.21 (t, *J* = 8.2 Hz, 1H), 6.65 (d, *J* = 8.3 Hz, 1H), 6.51 (d, *J* = 8.1 Hz, 1H), 5.82-5.73 (m, 1H), 4.88 (s, 2H), 4.58 (d, *J* = 5.6 Hz, 2H), 3.87 (d, *J* = 10.5 Hz, 3H), 2.98-2.85 (m, 1H), 2.66-2.56 (m, 2H), 2.12 (s, 1H).

### Example 8: N-((2-(2,6-Dioxopiperidin-3-yl)-1-oxo-1,2-dihydrophthalazin-6-yl)methyl)-6-methoxy-2H-chromene-3-carboxamide (Compound T-8)

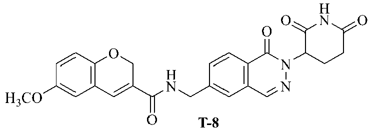

Synthesis of 6-methoxy-2H-chromene-3-carboxylic acid: With reference to the synthesis method for intermediate-3, 5-methoxysalicylaldehyde was used as the starting material to prepare the compound, with a two-step yield of 58.6%. HRMS (ESI⁻):(M - H)⁻ found 205.0511.

Synthesis of compound T-8: With reference to the synthesis method for compound T-1, 6-methoxy-2H-chromene-3-carboxylic acid was used as the starting material to prepare T-8 (a white solid, 0.06 g, yield: 40.8%, purity: 96.13%). ¹H NMR (300 MHz, DMSO-*d*₆) *δ* (ppm) 11.05 (s, 1H), 9.03 (t, *J* = 5.8 Hz, 1H), 8.56 (s, 1H), 8.26 (d, *J* = 8. Hz, 1H), 7.82 (d, *J* = 8.7 Hz, 2H), 7.36 (s, 1H), 7.00 (d, *J* = 8.0 Hz, 1H), 6.92 (t, *J* = 8.1 Hz, 1H), 6.74 (d, *J* = 6.3 Hz, 1H), 5.91-5.79 (m, 1H), 4.90 (s, 2H), 4.60 (d, *J* = 5.6 Hz, 2H), 3.79 (s, 3H), 2.98-2.87 (m, 1H), 2.64-2.55 (m, 2H), 2.14-2.09 (m, 1H).

### Example 9: N-((2-(2,6-Dioxopiperidin-3-yl)-1-oxo-1,2-dihydrophthalazin-6-yl)methyl)-8-(trifluoromethoxy)-2H-chromene-3-carboxamide (Compound T-9)

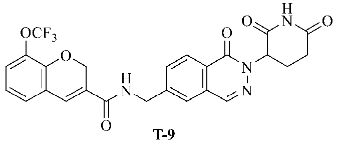

Synthesis of 8-trifluoromethoxy-2H-chromene-3-carboxylic acid: With reference to the synthesis method for intermediate-3, 2-hydroxy-3-trifluoromethoxybenzaldehyde was used as the starting material to prepare the compound, with a two-step yield of 48.2%. HRMS (ESI⁻):(M - H)⁻ found 259.0220.

Synthesis of compound T-9: With reference to the synthesis method for compound T-1, 8-trifluoromethoxy-2H-chromene-3-carboxylic acid was used as the starting material to prepare T-9 (a white solid, 0.09 g, yield: 54.9%, purity: 98.93%). ¹H NMR (300 MHz, DMSO-*d*₆) *δ* (ppm) 11.04 (s, 1H), 9.05 (t, *J* = 6.1 Hz, 1H), 8.49 (s, 1H), 8.26 (d, *J* = 8.1 Hz, 1H), 7.85 (d, *J* = 9.5 Hz, 2H), 7.40 (s, 1H), 7.31 (t, *J* = 8.2 Hz, 2H), 7.04 (t, *J* = 7.9 Hz, 1H), 5.85-5.79 (m, 1H), 5.06 (s, 2H), 4.62 (d, *J* = 5.3 Hz, 2H), 3.00-2.87 (m, 1H), 2.65-2.54 (m, 2H), 2.15-2.11 (m, 1H).

### Example 10: N-((2-(2,6-Dioxopiperidin-3-yl)-1-oxo-1,2-dihydrophthalazin-6-yl)methyl)-6-(trifluoromethoxy)-2H-chromene-3-carboxamide (Compound T-10)

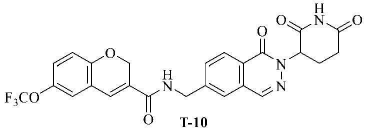

Synthesis of 6-trifluoromethoxy-2H-chromene-3-carboxylic acid: With reference to the synthesis method for intermediate-3, 5-trifluoromethoxysalicylaldehyde was used as the starting material to prepare the compound, with a two-step yield of 62.3%. HRMS (ESI⁻):(M - H)⁻ found 259.0228.

Synthesis of compound T-10: Through the synthesis method for compound T-1, 6-trifluoromethoxy-2H-chromene-3-carboxylic acid was used as the starting material to prepare A-10 (a white solid, 0.12 g, yield: 73.3%, purity: 99.89%). ¹H NMR (300 MHz, DMSO-*d*₆) *δ* (ppm) 11.05 (s, 1H), 9.05 (t, *J* = 5.9 Hz, 1H), 8.49 (s, 1H), 8.26 (d, *J* = 8.0 Hz, 1H), 7.84 (d, *J* = 8.9 Hz, 2H), 7.36 (s, 1H), 7.30 (s, 1H), 7.25 (d, *J* = 8.7 Hz, 1H), 6.97 (d, *J* = 8.7 Hz, 1H), 5.84-5.79 (m, 1H), 5.01 (s, 2H), 4.61 (d, *J* = 5.5 Hz, 2H), 2.99-2.87 (m, 1H), 2.64-2.54 (m, 2H), 2.16-2.10 (m, 1H).

### Example 11: N-((2-(2,6-Dioxopiperidin-3-yl)-1-oxo-1,2-dihydrophthalazin-6-yl)methyl)-8-methyl-2H-chromene-3-carboxamide (Compound T-11)

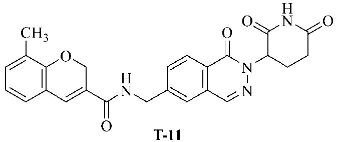

Synthesis of 8-methyl-2H-chromene-3-carboxylic acid: With reference to the synthesis method for intermediate-3, 2-hydroxy-3-methylbenzaldehyde was used as the starting material to prepare the compound, with a two-step yield of 60.2%. HRMS (ESI⁻):(M - H)⁻ found 189.0545. Synthesis of compound T-11: With reference to the synthesis method for compound T-1, 8-methyl-2H-chromene-3-carboxylic acid was used as the starting material to prepare T-11 (a white solid, 0.07 g, yield: 49.2%, purity: 98.35%). ¹H NMR (300 MHz, DMSO-*d*₆) *δ* (ppm) 11.05 (s, 1H), 9.03 (t, *J* = 6.2 Hz, 1H), 8.38 (s, 1H), 8.19 (d, *J* = 8.2 Hz, 1H), 7.82 (d, *J* = 6.8 Hz, 2H), 7.34 (s, 1H), 7.01 (d, *J* = 8.0 Hz, 1H), 6.82 (t, *J* = 9.4 Hz, 1H), 6.83 (d, *J* = 10.2 Hz, 1H), 5.86-5.78 (m, 1H), 4.94 (s, 2H), 4.33 (d, *J* = 3.4 Hz, 2H), 2.99-2.89 (m, 1H), 2.71-2.63 (m, 2H), 2.18 (s, 3H), 2.14-2.09 (m, 1H).

### Example 12: N-((2-(2,6-Dioxopiperidin-3-yl)-1-oxo-1,2-dihydrophthalazin-6-yl)methyl)-6-methyl-2H-chromene-3-carboxamide (Compound T-12)

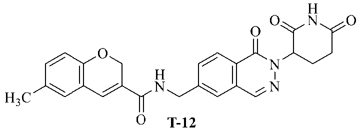

Synthesis of 6-methyl-2H-chromene-3-carboxylic acid: With reference to the synthesis method for intermediate-3, 5-methylsalicylaldehyde was used as the starting material to prepare the compound, with a two-step yield of 57.6%. HRMS (ESI⁻):(M - H)⁻ found 189.0548.

Synthesis of compound T-12: With reference to the synthesis method for compound T-1, 6-methyl-2H-chromene-3-carboxylic acid was used as the starting material to prepare T-12 (a white solid, 0.07 g, yield: 49.2%, purity: 98.96%). ¹H NMR (300 MHz, DMSO-*d*₆) *δ* (ppm) 11.00 (s, 1H), 8.97 (s, 1H), 8.55 (s, 1H), 8.13 (d, *J* = 9.7 Hz, 1H), 7.81 (d, *J* = 6.5 Hz, 2H), 7.33 (s, 1H), 7.02 (d, *J* = 10.0 Hz, 1H), 6.79 (t, *J* = 8.2 Hz, 1H), 6.83 (d, *J* = 10.2 Hz, 1H), 5.87-5.78 (m, 1H), 4.92 (s, 2H), 4.31 (d, *J* = 3.5 Hz, 2H), 2.98-2.87 (m, 1H), 2.72-2.64 (m, 2H), 2.17 (s, 3H), 2.12-2.07 (m, 1H).

### Example 13: 6-Chloro-N-((2-(2,6-dioxopiperidin-3-yl)-1-oxo-1,2-dihydrophthalazin-6-yl)methyl)-2H-chromene-3-carboxamide (Compound T-13)

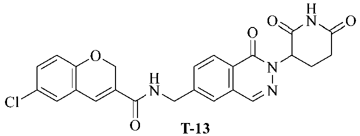

Synthesis of 6-chloro-2H-chromene-3-carboxylic acid: With reference to the synthesis method for intermediate-3, 5-chlorosalicylaldehyde was used as the starting material to prepare the compound, with a two-step yield of 68.3%. HRMS (ESI⁻):(M - H)⁻ found 209.0019.

Synthesis of compound T-13: With reference to the synthesis method for compound T-1, 6-chloro-2H-chromene-3-carboxylic acid was used as the starting material to prepare A-6 (a white solid, 0.06 g, yield: 40.4%, purity: 96.87%). ¹H NMR (300 MHz, DMSO-*d*₆) *δ* (ppm) 11.05 (s, 1H), 9.02 (s, 1H), 8.47 (s, 1H), 8.26 (d, *J* = 9.0 Hz, 1H), 7.83 (d, *J* = 10.7 Hz, 2H), 7.41 (s, 1H), 7.29 (s, 1H), 7.21 (d, *J* = 7.4 Hz, 1H), 6.91 (d, *J* = 7.3 Hz, 1H), 5.86-5.72 (m, 1H), 5.03 (s, 2H), 4.59 (d, *J* = 6.9 Hz, 2H), 2.97-2.86 (m, 1H), 2.64-2.54 (m, 2H), 2.19-2.11 (m, 1H).

### Example 14: 6-Bromo-N-((2-(2,6-dioxopiperidin-3-yl)-1-oxo-1,2-dihydrophthalazin-6-yl)methyl)-2H-chromene-3-carboxamide (Compound T-14)

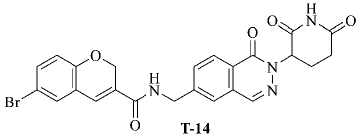

Synthesis of 6-bromo-2H-chromene-3-carboxylic acid: With reference to the synthesis method for intermediate-3, 5-bromosalicylaldehyde was used as the starting material to prepare the compound, with a two-step yield of 62.5%. HRMS (ESI⁻):(M - H)⁻ found 252.9513.

Synthesis of compound T-14: With reference to the synthesis method for compound T-1, 6-bromo-2H-chromene-3-carboxylic acid was used as the starting material to prepare A-6 (a white solid, 0.07 g, yield: 43.1%, purity: 97.03%). ¹H NMR (300 MHz, DMSO-*d*6) *δ* (ppm) 11.05 (s, 1H), 9.03 (s, 1H), 8.47 (s, 1H), 8.27 (d, *J* = 9.5 Hz, 1H), 7.82 (d, *J* = 9.7 Hz, 2H), 7.38 (s, 1H), 7.27 (s, 1H), 7.19 (d, *J* = 7.4 Hz, 1H), 6.90 (d, *J* = 7.4 Hz, 1H), 5.86-5.72 (m, 1H), 5.04 (s, 2H), 4.58 (d, *J* = 6.9 Hz, 2H), 2.97-2.87 (m, 1H), 2.63-2.54 (m, 2H), 2.19-2.11 (m, 1H).

### Example 15: N-((2-(2,6-Dioxopiperidin-3-yl)-1-oxo-1,2-dihydrophthalazin-6-yl)methyl)-5-(trifluoromethyl)-2H-chromene-3-carboxamide (Compound T-15)

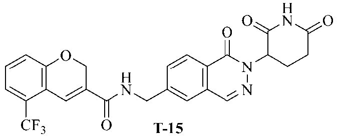

Synthesis of 5-(trifluoromethyl)-2H-chromene-3-carboxylic acid: With reference to the synthesis method for intermediate-3, 6-trifluoromethylsalicylaldehyde was used as the starting material to prepare the compound, with a two-step yield of 58.3%. HRMS (ESI⁻):(M - H)⁻ found 243.0279.

Synthesis of compound T-15: With reference to the synthesis method for compound T-1, 5-(trifluoromethyl)-2H-chromene-3-carboxylic acid was used as the starting material to prepare T-15 (a white solid, 0.06 g, yield: 37.8%, purity: 98.83%). ¹H NMR (300 MHz, DMSO-*d*₆) *δ* (ppm) 11.05 (s, 1H), 9.20 (t, *J* = 3.1 Hz, 1H), 8.46 (s, 1H), 8.25 (d, *J* = 8.1 Hz, 1H), 7.84 (d, *J* = 8.3 Hz, 2H), 7.44 (t, *J* = 8.8 Hz, 2H), 7.36 (d, *J* = 7.5 Hz, 1H), 7.21 (d, *J* = 8.0 Hz, 1H), 5.80-5.74 (m, 1H), 5.01 (s, 2H), 4.61 (d, *J* = 5.6 Hz, 2H), 2.96-2.84 (m, 1H), 2.68-2.57 (m, 2H), 2.16-2.12 (m, 1H).

### Example 16: N-((2-(2,6-Dioxopiperidin-3-yl)-1-oxo-1,2-dihydrophthalazin-6-yl)methyl)-6-vinyl-2H-chromene-3-carboxamide (Compound T-16)

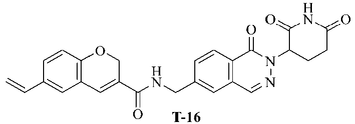

Synthesis of 6-vinyl-2H-chromene-3-carboxylic acid: With reference to the synthesis method for intermediate-3, 5-vinylsalicylaldehyde was used as the starting material to prepare the compound, with a two-step yield of 56.8%. HRMS (ESI⁻):(M - H)⁻ found 201.0568.

Synthesis of compound T-16: With reference to the synthesis method for compound T-1, 6-vinyl-2H-chromene-3-carboxylic acid was used as the starting material to prepare T-16 (a white solid, 0.08 g, yield: 54.9%, purity: 96.29%). ¹H NMR (300 MHz, DMSO-*d*₆) *δ* (ppm) 11.06 (s, 1H), 8.99 (s, 1H), 8.54 (s, 1H), 8.19 (d, *J* = 3.6 Hz, 1H), 7.99 (d, *J* = 5.0 Hz, 1H), 7.74 (s, 1H), 7.43-7.37 (m, 1H), 7.21 (t, *J* = 9.3 Hz, 1H), 6.83 (d, *J* = 4.0 Hz, 1H), 6.64 (t, *J* = 9.7 Hz, 1H), 6.21 (s, 1H), 5.85-5.79 (m, 1H), 5.76 (d, *J* = 6.9 Hz, 1H), 5.19 (d, *J* = 6.6 Hz, 1H), 5.06 (s, 2H), 4.62 (d, *J* = 5.3 Hz, 2H), 2.96-2.84 (m, 1H), 2.62-2.51 (m, 2H), 2.21-2.15 (m, 1H).

### Example 17: 6-(tert-Butyl)-N-((2-(2,6-dioxopiperidin-3-yl)-1-oxo-1,2-dihydrophthalazin-6-yl)methyl)-2H-chromene-3-carboxamide (Compound T-17)

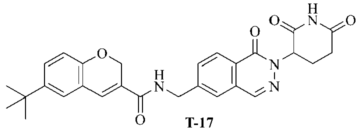

Synthesis of 6-(tert-butyl)-2H-chromene-3-carboxylic acid: With reference to the synthesis method for intermediate-3, 5-tert-butylsalicylaldehyde was used as the starting material to prepare the compound, with a two-step yield of 47.6%. HRMS (ESI⁻):(M - H)⁻ found 231.1023. Synthesis of compound T-17: With reference to the synthesis method for compound T-1, 6-(tert-butyl)-2H-chromene-3-carboxylic acid was used as the starting material to prepare T-17 (a white solid, 0.07 g, yield: 45.1%, purity: 98.93%). ¹H NMR (300 MHz, DMSO-*d*₆) *δ* (ppm) 11.05 (s, 1H), 9.03 (s, 1H), 8.64 (s, 1H), 8.19 (d, *J* = 6.9 Hz, 1H), 8.01 (d, *J* = 7.5 Hz, 1H), 7.83 (s, 1H), 7.46 (d, *J* = 9.0 Hz, 1H), 7.13 (d, *J* = 7.1 Hz, 1H), 7.01 (s, 1H), 6.64 (t, *J* = 8.3 Hz, 1H), 5.85-5.79 (m, 1H), 5.06 (s, 2H), 4.72 (d, *J* = 5.2 Hz, 2H), 2.93-2.81 (m, 1H), 2.61-2.51 (m, 2H), 2.20-2.15 (m, 1H), 1.25 (s, 9H).

### Example 18: N-((2-(2,6-Dioxopiperidin-3-yl)-1-oxo-1,2-dihydrophthalazin-6-yl)methyl)-6-phenyl-2H-chromene-3-carboxamide (Compound T-18)

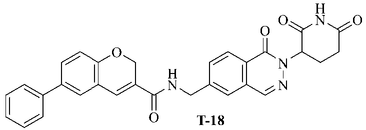

Synthesis of 6-phenyl-2H-chromene-3-carboxylic acid: With reference to the synthesis method for intermediate-3, 5-phenylsalicylaldehyde was used as the starting material to prepare the compound, with a two-step yield of 53.2%. HRMS (ESI⁻):(M - H)⁻ found 251.0718.

Synthesis of compound T-18: With reference to the synthesis method for compound T-1, 6-phenyl-2H-chromene-3-carboxylic acid was used as the starting material to prepare T-18 (a white solid, 0.05 g, yield: 31.0%, purity: 96.54%). ¹H NMR (300 MHz, DMSO-*d*₆) *δ* (ppm) 11.03 (s, 1H), 8.90 (t, *J* = 6.2 Hz, 1H), 8.55 (s, 1H), 8.13 (d, *J* = 7.3 Hz, 1H), 7.98 (s, 1H), 7.76-7.63 (m, 4H), 7.52-7.41 (m, 5H), 7.01 (d, *J* = 8.3 Hz, 1H), 5.80-5.74 (m, 1H), 5.01 (s, 2H), 4.67 (d, *J* = 5.6 Hz, 2H), 2.91-2.83 (m, 1H), 2.62-2.54 (m, 2H), 2.19-2.11 (m, 1H).

### Example 19: N-((2-(2,6-Dioxopiperidin-3-yl)-1-oxo-1,2-dihydrophthalazin-6-yl)methyl)-6,8-difluoro-2H-chromene-3-carboxamide (Compound T-19)

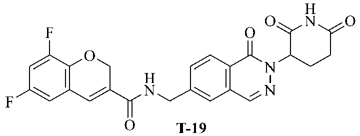

Synthesis of 6,8-difluoro-2H-chromene-3-carboxylic acid: With reference to the synthesis method for intermediate-3, 3,5-difluorosalicylaldehyde was used as the starting material to prepare the compound, with a two-step yield of 52.4%. HRMS (ESI⁻):(M - H)⁻ found 211.0220. Synthesis of compound T-19: With reference to the synthesis method for compound T-1, 6,8-difluoro-2H-chromene-3-carboxylic acid was used as the starting material to prepare T-19 (a white solid, 0.06 g, yield: 40.3%, purity: 96.93%). ¹H NMR (300 MHz, DMSO-*d*₆) *δ* (ppm) 11.01 (s, 1H), 8.95 (s, 1H), 8.20 (s, 1H), 7.94 (d, *J* = 8.5 Hz, 1H), 7.77 (t, *J* = 14.3 Hz, 2H), 7.38 (t, *J* = 9.6 Hz, 1H), 7.15 (d, *J* = 8.9 Hz, 1H), 5.86-5.80 (m, 1H), 5.11 (s, 2H), 4.59 (d, *J* = 6.4 Hz, 2H), 2.87-2.74 (m, 1H), 2.75-2.65 (m, 2H), 2.18-2.10 (m, 1H).

### Example 20: 6-Chloro-N-((2-(2,6-dioxopiperidin-3-yl)-1-oxo-1,2-dihydrophthalazin-6-yl)methyl)-7-methyl-2H-chromene-3-carboxamide (Compound T-20)

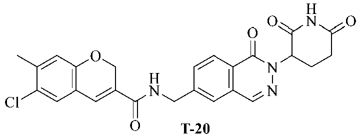

Synthesis of 6-chloro-7-methyl-2H-chromene-3-carboxylic acid: With reference to the synthesis method for intermediate-3, 5-chloro-4-methylsalicylaldehyde was used as the starting material to prepare the compound, with a two-step yield of 54.6%. HRMS (ESI⁻):(M - H)⁻ found 223.0158. Synthesis of compound T-20: With reference to the synthesis method for compound T-1, 6-chloro-7-methyl-2H-chromene-3-carboxylic acid was used as the starting material to prepare T-20 (a white solid, 0.08 g, yield: 52.4%, purity: 98.43%). ¹H NMR (300 MHz, DMSO-*d*₆) *δ* (ppm) 11.01 (s, 1H), 8.95 (t, *J* = 7.2 Hz, 1H), 8.10 (s, 1H), 7.78 (s, 1H), 7.48 (d, *J* = 6.8 Hz, 2H), 7.14 (d, *J* = 9.6 Hz, 1H), 7.02 (s, 1H), 6.62 (s, 1H), 5.85-5.80 (m, 1H), 5.08 (s, 2H), 4.59 (d, *J* = 3.4 Hz, 2H), 2.87-2.74 (m, 4H), 2.62-2.57 (m, 2H), 2.21-2.13 (m, 1H).

### Example 21: N-((2-(2,6-Dioxopiperidin-3-yl)-1-oxo-1,2-dihydrophthalazin-6-yl)methyl)chromane-3-carboxamide (Compound T-21)

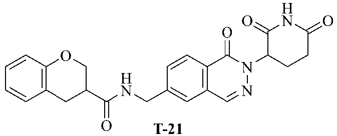

Synthesis of chroman-3-carboxylic acid: With reference to the synthesis method for intermediate-3, salicylaldehyde was used as the starting material to prepare the compound, with a three-step yield of 49.3%. HRMS (ESI⁻):(M - H)⁻ found 177.0559.

Synthesis of compound T-21: With reference to the synthesis method for compound T-1, chroman-3-carboxylic acid was used as the starting material to prepare T-21 (a white solid, 0.08 g, yield: 57.8%, purity: 98.13%). ¹H NMR (300 MHz, DMSO-*d*₆) *δ* (ppm) 11.03 (s, 1H), 9.02 (s, 1H), 8.78 (s, 1H), 8.49 (d, *J* = 6.3 Hz, 1H), 8.19 (t, *J* = 9.8 Hz, 1H), 7.84 (d, *J* = 9.8 Hz, 1H), 7.43 (d, *J* = 8.2 Hz, 1H), 7.02-6.91 (m, 3H), 5.84-5.78 (m, 1H), 5.06 (s, 2H), 4.62 (d, *J* = 5.3 Hz, 2H), 2.88-2.73 (m, 4H), 2.69-2.54 (m, 2H), 2.22-2.14 (m, 1H).

### Example 22: N-((2-(2,6-Dioxopiperidin-3-yl)-1-oxo-1,2-dihydrophthalazin-6-yl)methyl)-6-fluorochromane-3-carboxamide (Compound T-22)

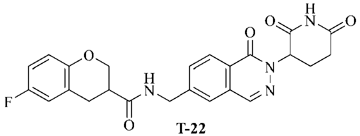

Synthesis of 6-fluorochromane-3-carboxylic acid: With reference to the synthesis method for intermediate-3, 5-fluorosalicylaldehyde was used as the starting material to prepare the compound, with a three-step yield of 51.2%. HRMS (ESI⁻):(M - H)⁻ found 195.0468.

Synthesis of compound T-22: With reference to the synthesis method for compound T-1, 6-fluorochromane-3-carboxylic acid was used as the starting material to prepare T-22 (a white solid, 0.06 g, yield: 41.7%, purity: 98.03%). ¹H NMR (300 MHz, DMSO-*d*₆) *δ* (ppm) 11.03 (s, 1H), 9.03 (s, 1H), 8.76 (s, 1H), 8.49 (d, *J* = 7.2 Hz, 1H), 8.20 (t, *J* = 9.8 Hz, 1H), 7.79 (d, *J* = 9.7 Hz, 1H), 7.40 (d, *J* = 8.2 Hz, 1H), 7.01-6.91 (m, 2H), 5.85-5.79 (m, 1H), 5.08 (s, 2H), 4.65 (d, *J* = 5.6 Hz, 2H), 2.88-2.74 (m, 4H), 2.66-2.57 (m, 2H), 2.23-2.15 (m, 1H).

### Example 23: N-((2-(2,6-Dioxopiperidin-3-yl)-1-oxo-1,2-dihydrophthalazin-6-yl)methyl)-6-(trifluoromethoxy)chromane-3-carboxamide (Compound T-23)

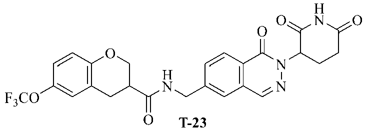

Synthesis of 6-trifluoromethoxychromane-3-carboxylic acid: With reference to the synthesis method for intermediate-3, 5-trifluoromethoxysalicylaldehyde was used as the starting material to prepare the compound, with a three-step yield of 50.8%. HRMS (ESI⁻):(M - H)⁻ found 261.0386.

Synthesis of compound T-23: With reference to the synthesis method for compound T-1, 6-trifluoromethoxychromane-3-carboxylic acid was used as the starting material to prepare T-23 (a white solid, 0.07 g, yield: 42.6%, purity: 99.10%). ¹H NMR (300 MHz, DMSO-*d*₆) *δ* (ppm) 11.04 (s, 1H), 9.13 (s, 1H), 8.54 (s, 1H), 8.12 (d, *J* = 8.2 Hz, 1H), 7.99 (t, *J* = 9.8 Hz, 1H), 7.52 (d, *J* = 9.7 Hz, 1H), 6.95-6.82 (m, 3H), 5.81-5.75 (m, 1H), 5.04 (s, 2H), 4.61 (d, *J* = 5.3 Hz, 2H), 2.69-2.62 (m, 4H), 2.58-2.53 (m, 2H), 2.19-2.11 (m, 1H).

### Example 24: N-((2-(2,6-Dioxopiperidin-3-yl)-1-oxo-1,2-dihydrophthalazin-6-yl)methyl)-1,2,3,4-tetrahydroquinoline-3-carboxamide (Compound T-24)

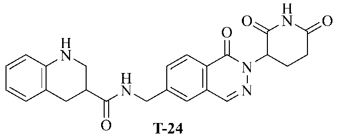

With reference to the synthesis method for compound T-1, 1,2,3,4-tetrahydroquinoline-3-carboxylic acid was used as the starting material to prepare T-24 (a white solid, 0.09 g, yield: 65.2%, purity: 98.63%). ¹H NMR (300 MHz, DMSO-*d*₆) *δ* (ppm) 11.06 (s, 1H), 8.97 (s, 1H), 8.62 (s, 1H), 8.13 (d, *J* = 8.4 Hz, 1H), 7.78 (d, *J* = 7.6 Hz, 1H), 7.31 (t, *J* = 10.2 Hz, 1H), 7.12 (t, *J* = 9.6 Hz, 1H), 6.91 (d, *J* = 12.8 Hz, 1H), 6.72 (t, *J* = 9.65 Hz, 1H), 6.48-6.40 (m, 2H), 5.87-5.81 (m, 1H), 5.08 (s, 2H), 4.65 (s, 2H), 2.93-2.79 (m, 4H), 2.69-2.55 (m, 2H), 2.21-2.14 (m, 1H).

### Example 25: N-(2-(2,6-Dioxopiperidin-3-yl)-1-oxo-1,2-dihydrophthalazin-6-yl)-2H-chromene-3-carboxamide (Compound T-25)

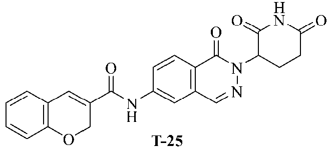

With reference to the synthesis method for compound T-1, intermediate-2 was used as the starting material to prepare T-25 (a white solid, 0.07 g, yield: 50.8%, purity: 99.03%). ¹H NMR (300 MHz, DMSO-*d*₆) *δ* (ppm) 11.06 (s, 1H), 9.12 (s, 1H), 8.43 (s, 1H), 8.19 (d, *J* = 9.8 Hz, 1H), 8.02 (s, 1H), 7.92 (s, 2H), 7.39 (s, 1H), 7.16 (t, *J* = 7.4 Hz, 1H), 7.12-7.06 (m, 2H), 5.86-5.79 (m, 1H), 5.09 (s, 2H), 2.99-2.85 (m, 1H), 2.64-2.55 (m, 2H), 2.16-2.12 (m, 1H).

### Example 26: N-(2-(2,6-Dioxopiperidin-3-yl)-1-oxo-1,2-dihydrophthalazin-6-yl)-6-fluoro-2H-chromene-3-carboxamide (Compound T-26)

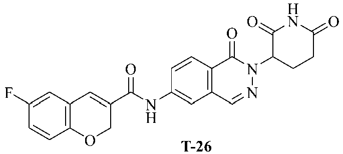

With reference to the synthesis method for compound T-1, intermediate-2 was used as the starting material to prepare T-26 (a white solid, 0.09 g, yield: 62.7%, purity: 98.27%). ¹H NMR (300 MHz, DMSO-*d*₆) *δ* (ppm) 11.02 (s, 1H), 9.11 (s, 1H), 8.43 (s, 1H), 8.21 (d, *J* = 9.9 Hz, 1H), 7.94 (d, *J* = 9.5 Hz, 1H), 7.58-7.49 (m, 2H), 7.23 (t, *J* = 10.2 Hz, 1H), 7.06-6.97 (m, 2H), 5.82-5.76 (m, 1H), 5.05 (s, 2H), 2.96-2.83 (m, 1H), 2.60-2.50 (m, 2H), 2.12-2.08 (m, 1H).

### Example 27: N-(2-(2,6-Dioxopiperidin-3-yl)-1-oxo-1,2-dihydrophthalazin-6-yl)-6-(trifluoromethoxy)-2H-chromene-3-carboxamide (Compound T-27)

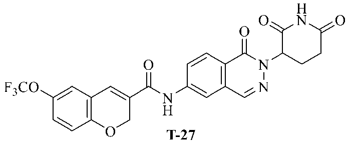

With reference to the synthesis method for compound T-1, intermediate-2 was used as the starting material to prepare T-27 (a white solid, 0.08 g, yield: 48.60%, purity: 97.93%). ¹H NMR (300 MHz, DMSO-*d*₆) *δ* (ppm) 11.01 (s, 1H), 9.19 (s, 1H), 8.32 (d, *J* = 8.9 Hz, 1H), 8.26 (d,*J* = 6.7 Hz, 1H), 8.03 (s, 1H), 7.95-7.86 (m, 2H), 7.21-7.14 (m, 1H), 7.03-6.92 (m, 2H), 5.85-5.79 (m, 1H), 5.08 (s, 2H), 2.99-2.87 (m, 1H), 2.64-2.54 (m, 2H), 2.16-2.10 (m, 1H).

### Example 28: N-(2-(2,6-Dioxopiperidin-3-yl)-1-oxo-1,2-dihydrophthalazin-6-yl)-8-(trifluoromethoxy)-2H-chromene-3-carboxamide (Compound T-28)

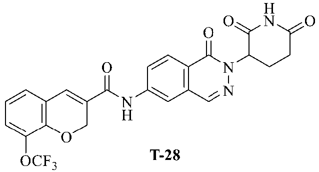

With reference to the synthesis method for compound T-1, intermediate-2 was used as the starting material to prepare T-28 (a white solid, 0.09 g, yield: 54.7%, purity: 99.32%). ¹H NMR (300 MHz, DMSO-*d*₆) *δ* (ppm) 11.06 (s, 1H), 8.94 (s, 1H), 8.47 (s, 1H), 8.27 (d, *J* = 7.3 Hz, 1H), 7.95 (d, *J* = 9.8 Hz, 1H), 7.76-7.72 (m, 2H), 7.21 (t, *J* = 11.6 Hz, 1H), 7.04 (t, *J* = 9.3 Hz, 1H), 6.92-6.87 (m, 1H), 5.80-5.74 (m, 1H), 5.02 (s, 2H), 2.92-2.85 (m, 1H), 2.65-2.54 (m, 2H), 2.14-2.11 (s, 1H).

### Biological Test Example 1: Study on Degradation of GSPT1 in KG-1 Cells

KG-1 cells were grown in an IMDM culture medium (containing 20% FBS). After centrifugation and counting, the cell concentration was adjusted. The cells were plated onto 6-well plates at 10⁶ cells/well, with 1350 µL per well. 150 µL of DMSO and the test compounds of the present disclosure (compound concentration: 200 nM) were added. The plates were incubated in an incubator at 37 °C with 5% CO2 for 4 h. The cells were centrifuged, and the culture medium was discarded. PBS was added to wash the cells and then discarded. Whole cell lysates were prepared using a protease inhibitor cocktail (100×, Fude Biological), a protein phosphatase inhibitor cocktail (100×, Fude Biological), Super Nuclease (Beyotime), and a high-intensity RIPA lysis buffer (Thermo Fisher) and placed on ice for 30 min. After centrifugation, the cell debris precipitates were discarded, and the whole cell lysate supernatants were collected and transferred to new centrifuge tubes. After a BCA protein assay, samples were prepared using a 5× loading buffer (Thermo Fisher). The samples were subjected to protein electrophoresis separation in a 4-20% precast gel (SDS-PAGE gel), transferred to a PVDF membrane, blocked with 5% NFDM/TBST at room temperature for 1 h, incubated overnight with primary antibodies at 4 °C, and incubated with a secondary antibody at room temperature for 2 h the next day. Signals were detected using a MINICHEMITM imaging system.

Primary antibodies:
Anti-GSPT1: Abcam ab234433
Anti-beta actin: Fude Biological FD0060

Secondary antibody:
Anti-rabbit peroxidase-linked secondary antibody: Fude Biological FDG007

The degradation effects of the compounds on GSPT1 protein are shown in Table 1, where A represents a GSPT1 protein degradation percentage of no less than 70%, B represents a degradation percentage of less than 70% but no less than 30%, and C represents a degradation percentage of less than 30%.

**Table 1. The degradation of GSPT1 protein by compounds**

| Compound | GSPT1 degradation | Compound | GSPT1 degradation |
|---|---|---|---|
| T-1 | B | T-15 | A |
| T-2 | A | T-16 | C |
| T-3 | B | T-17 | A |
| T-4 | B | T-18 | A |
| T-5 | B | T-19 | B |
| T-6 | B | T-20 | B |
| T-7 | A | T-21 | B |
| T-8 | B | T-22 | B |
| T-9 | B | T-23 | A |
| T-10 | A | T-24 | A |
| T-11 | B | T-25 | C |
| T-12 | B | T-26 | C |
| T-13 | B | T-27 | C |
| T-14 | B | T-28 | B |

As can be seen from Table 1, some of the phthalazinone compounds of the present disclosure exhibit a highly significant degradation effect on the GSPT1 protein.

Biological Test Example 2: Antiproliferative Activity Against KG-1 Cells

KG-1 cells in the logarithmic growth phase were diluted in a culture medium (RPMI + 10% FBS) and plated onto 96-well plates at 5000 cells/well, and the plates were incubated in an incubator at 37 °C with 5% CO₂ for 24 h. 10 mM stock solutions of the test compounds of the present disclosure in DMSO were prepared and diluted with the culture medium to set 9 concentration gradients. 3 replicate wells were set for each concentration. After compound addition, the plates were incubated in an incubator at 37 °C with 5% CO₂ for 72 h. The cell proliferation was assessed using the CCK8 method. The cell growth inhibition IC₅₀ values of the compounds were determined using an enzyme labeling method. The IC₅₀ values were calculated using GraphPad Prism 7 software.

The antiproliferative activity of the compounds against KG-1 cells is shown in Table 2, where A represents IC₅₀ ≤ 30 nM, B represents 30 < IC₅₀ ≤ 300 nM, and C represents IC₅₀ > 300 nM.

**Table 2. The antiproliferative activity of compounds against KG-1 cells**

| Compound | IC₅₀ | Compound | IC₅₀ |
|---|---|---|---|
| T-1 | B | T-15 | A |
| T-2 | A | T-16 | C |
| T-3 | B | T-17 | A |
| T-4 | B | T-18 | A |
| T-5 | A | T-19 | B |
| T-6 | B | T-20 | B |
| T-7 | A | T-21 | B |
| T-8 | B | T-22 | A |
| T-9 | A | T-23 | A |
| T-10 | A | T-24 | B |
| T-11 | B | T-25 | B |
| T-12 | B | T-26 | B |
| T-13 | B | T-27 | B |
| T-14 | B | T-28 | B |

As can be seen from Table 2, some of the phthalazinone compounds of the present disclosure exhibit highly significant antiproliferative inhibitory activity against KG-1 cells.

As described above, while the present disclosure has been shown and described with reference to particular preferred examples, they are not to be construed as limitations on the present disclosure per se. Various changes can be made in its form and details without departing from the spirit and scope of the present disclosure defined by the appended claims.

## Claims

1. A compound represented by general formula I or a pharmaceutically acceptable salt, tautomer, mesomer, racemate, enantiomer, or diastereomer thereof: 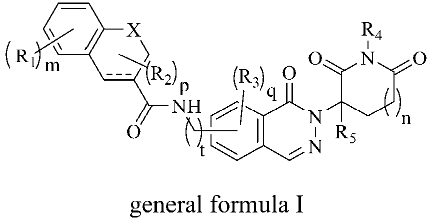 wherein:
- - - - represents a single or double bond;
X is selected from -CH₂-, -NH-, -O-, -S-, and -Se-;
each R₁ is independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, nitro, - C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, or heteroaryl is unsubstituted or optionally substituted with 1-3 Rₐ groups;
each R₂ is independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, nitro, - C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, or heteroaryl is unsubstituted or optionally substituted with 1-3 Rₐ groups;
each R₃ is independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, nitro, - C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, or heteroaryl is unsubstituted or optionally substituted with 1-3 Rₐ groups;
R₄ is selected from hydrogen, deuterium, C₁-C₁₂ alkyl, 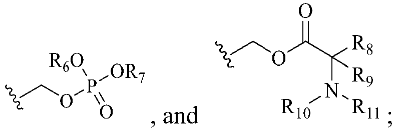
R₅ is selected from hydrogen, deuterium, and C₁-C₁₂ alkyl;
R₆, R₇, R₈, R₉, R₁₀, and R₁₁ are each independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, nitro, -C(O)NRₐR_{b}, -C(O)Rₐ, -C(O)ORₐ, -ORₐ, -OC(O)Rₐ, -OC(O)ORₐ, - OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, or heteroaryl is unsubstituted or optionally substituted with 1-3 R ₐ groups; Rₐ and R_{b} are each independently selected from hydrogen, C₁-C₆ alkyl, C₂-C₆ alkenyl, C₂-C₆ alkynyl, halogen, hydroxyl, cyano, nitro, benzyl, -C(O)NR_{c}R_{d}, -C(O)R_{c}, -C(O)OR_{c}, -OR_{c}, - OC(O)R_{c}, -OC(O)OR_{c}, -OC(O)NR_{c}R_{d}, -NR_{c}R_{d}, -SR_{c}, -S(O)R_{c}, -S(O)₂R_{c}, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, heteroaryl, or benzyl is unsubstituted or optionally substituted with 1-3 R_{c} groups;
R_{c} and R_{d} are each independently selected from hydrogen, halogen, carbonyl, hydroxyl, cyano, nitro, phenyl, benzyl, C₁-C₆ alkyl, halogenated C₁-C₆ alkyl, C₁-C₆ alkoxy, halogenated C₁-C₆ alkoxy, C₃-C₆ cycloalkyl, and halogenated C₃-C₆ cycloalkyl;
m, p, q, t, and n are each independently selected from 0, 1, 2, and 3.

2. The compound according to claim 1, wherein:
X is selected from -NH- and -O-.

3. The compound according to claim 1, wherein:
each R₁ is independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, hydroxyl, nitro, C₁-C₁₀ aryl, and -NRₐR_{b};
Rₐ and R_{b} are each independently selected from hydrogen, C₁-C₆ alkyl, and -C(O)R_{c};
R_{c} is selected from hydrogen, halogen, C₁-C₆ alkyl, halogenated C₁-C₆ alkyl, C₁-C₆ alkoxy, halogenated C₁-C₆ alkoxy, and C₃-C₆ cycloalkyl.

4. The compound according to claim 3, wherein:
each R₁ is independently selected from hydrogen, methyl, fluorine, chlorine, bromine, methoxy, trifluoromethyl, trifluoromethoxy, vinyl, tert-butyl, and phenyl.

5. The compound according to claim 1, wherein:
R₂ and R₃ are each independently selected from hydrogen.

6. The compound according to claim 1, wherein:
R₄ and R₅ are each independently selected from hydrogen.

7. The compound according to claim 1, wherein:
p, q, and n are each independently selected from 1.

8. The compound according to claim 1, wherein:
m is selected from 0, 1, and 2.

9. The compound according to claim 1, wherein:
t is selected from 0 and 1.

10. The compound according to claim 1, wherein the compound is selected from:

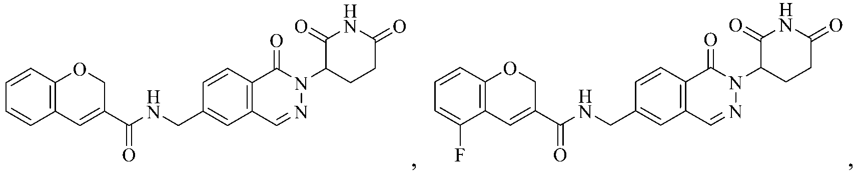

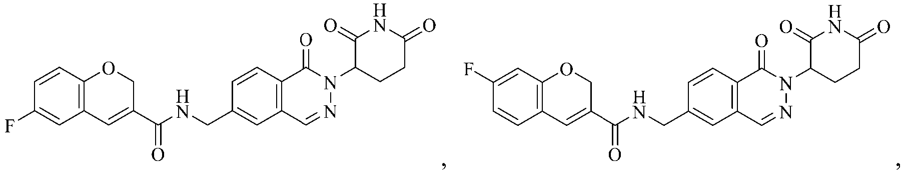

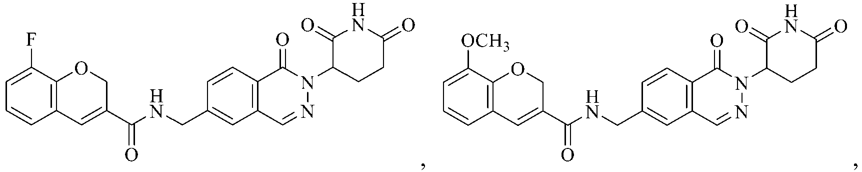

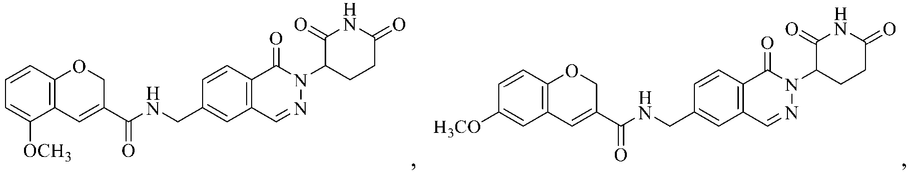

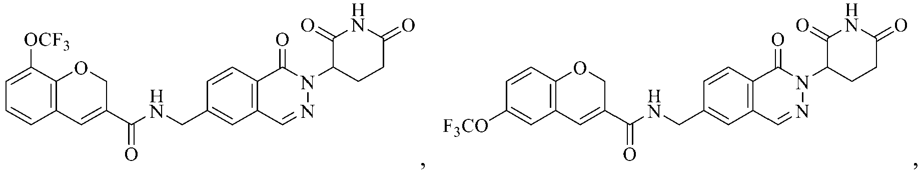

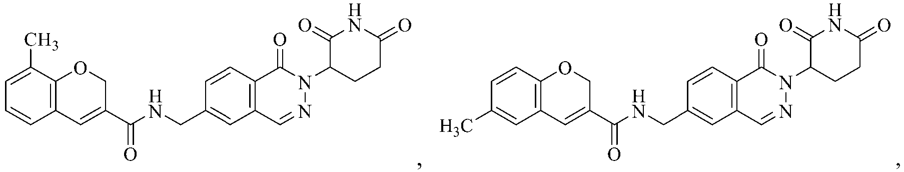

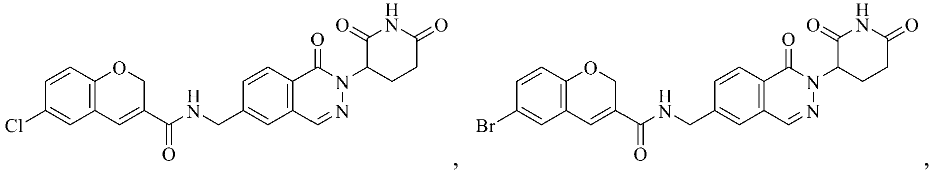

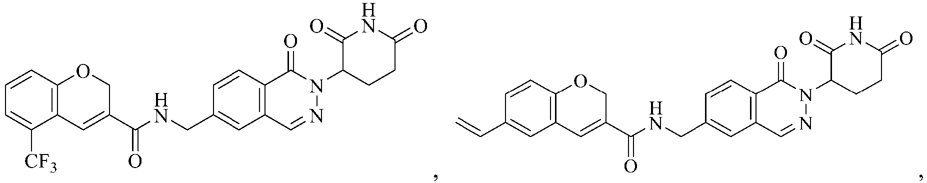

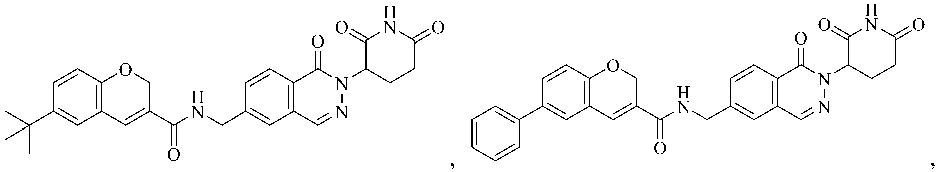

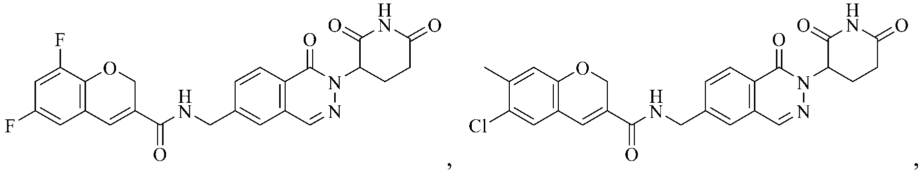

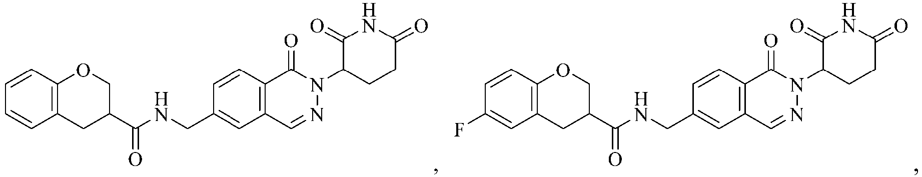

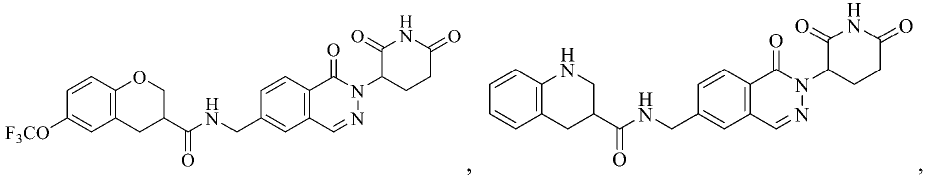

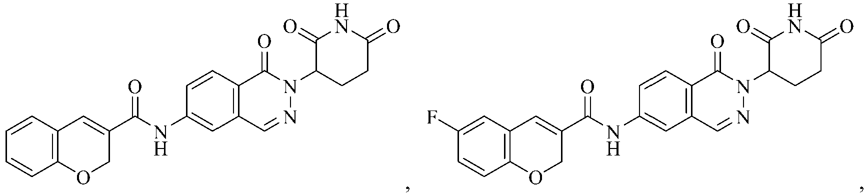

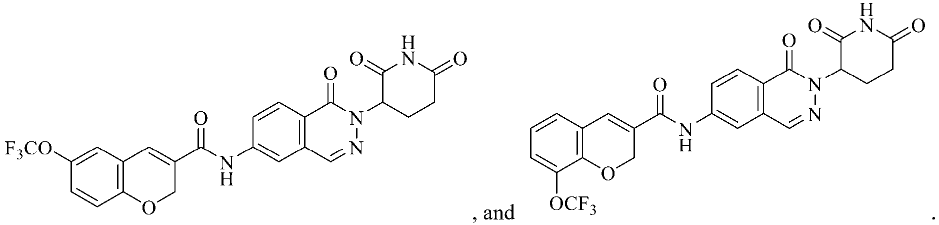

11. A pharmaceutical composition, comprising a therapeutically effective amount of the compound or the pharmaceutically acceptable salt, tautomer, mesomer, racemate, enantiomer, or diastereomer thereof according to any one of claims 1-10 and a pharmaceutically acceptable carrier or excipient.

12. Use of the compound according to any one of claims 1-10 in the preparation of a medicament for treating or preventing a disease related to mutation, unbalanced expression, allosterism, and dysfunction of GSPT1, IKZF1, IKZF2, IKZF3, CK1α, N-MYC, or C-MYC protein.

13. The use according to claim 12, wherein the related disease is cancer, a viral infection, aging, an immune disease, or a neurological disease.

14. The use according to claim 13, wherein the cancer is selected from acute myeloid leukemia, liver cancer, acute lymphocytic leukemia, bladder cancer, bone cancer, brain cancer, breast cancer, cervical cancer, choriocarcinoma, chronic lymphocytic leukemia (CLL), chronic granulocytic leukemia (CML), colon cancer, esophageal cancer, gallbladder cancer, gastric cancer, gastrointestinal stromal tumor, head and neck cancer, Hodgkin lymphoma, laryngeal cancer, lung cancer, melanoma, mesothelioma, multiple myeloma, ovarian cancer, pancreatic cancer, prostate cancer, rectal cancer, renal cancer, skin cancer, testicular cancer, throat cancer, thyroid cancer, and uterine cancer.

15. The use according to claim 13, wherein the lung cancer is non-small cell lung cancer.

16. Use of the compound according to any one of claims 1-10 in the preparation of a GSPT1 degrader.
